# EUROPÄISCHE PATENTSCHRIFT

(11) **EP 3 313 479 B1**
(45) Veröffentlichungstag und Bekanntmachung des Hinweises auf die Patenterteilung: **04.08.2021**
(21) Anmeldenummer: 16730237.1
(22) Anmeldetag: 15.06.2016
(51) Int. Cl.: A61M 5/315, A61M 5/20, A61M 5/31, A61M 5/32, A61M 5/46, A61M 5/48

(54) **AUSSCHÜTTEINRICHTUNG MIT DÄMPFUNG**
DISCHARGE DEVICE WITH DAMPING
SYSTÈME DE DISTRIBUTION ÉQUIPÉ D'UN DISPOSITIF D'AMORTISSEMENT

(30) Priorität: 23.06.2015 CH 9042015; 22.02.2016 CH 2292016
(43) Veröffentlichungstag der Anmeldung: 02.05.2018
(73) Patentinhaber: Ypsomed AG, 3401 Burgdorf (CH)
(72) Erfinder: HOSTETTLER, Patrick, 3415 Hasle (CH); STREIT, Ursina, 3322 Schönbühl (CH); TSCHIRREN, Markus, 3400 Burgdorf (CH)
(74) Vertreter: Kleiner, Stefan
(86) Internationale Anmeldenummer: PCT/CH2016/000091
(87) Internationale Veröffentlichungsnummer: WO 2016/205962

(56) Entgegenhaltungen:
- EP-A1- 2 468 329
- WO-A1-2014/159017
- WO-A1-2015/185311
- WO-A2-2011/109205
- GB-A- 2 414 404
- US-B1- 7 901 377

## Beschreibung

Die vorliegende Patentanmeldung bezieht ihr Prioritätsdatum von der Patentanmeldung 00904/15 (CH), welche am 23.06.2015 beim Eidgenössischen Institut für Geistiges Eigentum in der Schweiz eingereicht worden ist.

Die Erfindung betrifft eine Injektionsvorrichtung sowie Verfahren zum Verabreichen eines flüssigen Produkts insbesondere eines hoch viskosen Medikaments. Im Besonderen betrifft die Erfindung eine verbesserte Antriebs- und Signalvorrichtung sowie ein Verfahren für eine solche Injektionsvorrichtung.

Der Begriff "Medikament" oder Produkt umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament oder Produkt umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Aus der WO2014/146210 ist ein Autoinjektor bekannt, bei welchem am Ende der Injektion ein akustisches oder taktiles Signal erzeugt wird, um der benutzenden Person anzuzeigen, dass der Injektionsvorgang abgeschlossen ist. Bei dem vorgestellten Autoinjektor wird das Signal erzeugt, sobald und unmittelbar nachdem eine Kolbenstange den Stopfen einer vorbefüllten Spritze um eine bestimmte Distanz verschoben hat. Bei viskosen Medikamenten kann es bei dieser Anordnung sein, dass beim Auslösen des Endsignals, noch nicht die ganze Menge Medikament auch tatsächlich den Autoinjektor verlassen hat.

Aus der EP2542280B1 ist ein Autoinjektor bekannt, bei welchem die Spritze nach der Injektion zurück in den Autoinjektor gezogen wird. Der Rückzugsmechanismus wird ausgelöst, nachdem eine Kolbenstange den Stopfen der vorbefüllten Spritze um eine gewisse Distanz verschoben hat. Der Spritzenrückzug wird dabei nicht unmittelbar nach dem Erreichen der gewissen Distanz vollführt, sondern erst nach einer bestimmten Verzögerung. Unmittelbar nach dem Erreichen der gewissen Distanz wird durch die Bewegung der Kolbenstange ein Freigabeteil freigegeben, welches sich durch die Wirkung einer vorgespannten Torsionfeder zu drehen beginnt. Erst wenn sich das Freigabeteil um einen bestimmten Winkel gedreht hat, wird der Spritzenrückzug freigegeben. Die Drehung des Freigabeteils erzeugt also eine Verzögerung beim Spritzenrückzug. In der Schrift wird das Auslösen des Spritzenrückzug weiter verzögert, in dem die Rotation des Freigabeteils durch eine viskose Substanz gebremst wird. Die in der Schrift beschriebene Anordnung ist kompliziert und aufwendig. Sie hat den Nachteil, dass die Spritze vor und nach der Injektion bewegt werden muss.

Aus der WO2014/159017 ist ebenfalls ein Autoinjektor mit mehrteiliger Kolbenstange bekannt.

Es ist eine Aufgabe der Erfindung, eine Injektionsvorrichtung anzugeben, die eine sichere Einrichtung zum Ausschütten von hochviskosen Medikamenten aufweist und dabei kosteneffizient herstellbar ist.

Die Aufgabe wird mit der Ausschütteinrichtung, insbesondere einem Autoinjektor, nach Anspruch 1 gelöst. Vorteilhafte Weiterentwicklungen ergeben sich aus den abhängigen Ansprüchen, der Beschreibung und den Figuren.

Die Erfindung ist in Anspruch 1 definiert. Die abhängigen Ansprüche definieren sinnvolle Erweiterungen.

Beschrieben wird weiter ein Verfahren zum Verzögern eines Freigabemechanismus bei Verabreichung eines fluiden Produktes mit einer Ausschütteinrichtung wie im obigen Absatz beschrieben, wobei die Verabreichung des fluiden Produkt über die Auslösevorrichtung gestartet wird, wodurch die gespeicherte Energie der Triebfeder als Drehmoment auf die Gewindestange wirkt, so dass wenn Energie aus der Triebfeder freigesetzt wird, die Gewindestange mit einem Drehmoment beaufschlagt wird und in Drehung um die Längsachse versetzt werden kann, wobei, wenn sich beim Verbreichen die Gewindestange dreht, über die Gewindeverbindung zur inneren Kolbenstange auch die innere Kolbenstange bewegt wird, wobei die innere Kolbenstange in einer ersten Phase gegenüber dem Mechanikhalter über die Längsführung verdrehgesichert und verschiebbar gelagert ist und wobei die Verdrehsicherung für eine zweite Phase aufgehoben wird, nachdem die innere Kolbenstange) über eine gewisse Distanz in distale Richtung verschoben wird, wobei eine relative Bewegung der inneren Kolbenstange zur äusseren Kolbenstange in distale Richtung durch einen axialen Anschlag auf einer inneren Oberfläche der äusseren Kolbenstange begrenzt wird, wobei die Verschiebung der inneren Kolbenstange aufgrund des axialen Anschlages eine Verschiebung der äusseren Kolbenstange in distale Richtung und damit eine Verschiebung des Stopfens in distale Richtung und schlussendlich Ausschüttung von Produkt zur Folge hat, wobei die äussere Kolbenstange, welche über die Längsführung axial verschiebbar aber verdrehgesichert am Mechanikhalter angeordnet ist, wobei das Drehmoment in der zweiten Phase eine Drehung der inneren Kolbenstange relativ zur äusseren Kolbenstange zur Folge hat, welche durch das viskose Fluid gebremst wird und welche die mindestens eine Freigabestruktur in eine vorbestimmte Orientierung bringen kann, in welcher die freizugebende Struktur freigegeben wird.

In einem weiteren Aspekt wird beschrieben, dass das innere Volumen der Kavität durch eine lokale und auf dem Umfang verlaufende Verjüngung auf der inneren Kolbenstange und der der Verjüngung gegenüberliegenden Innenfläche der äusseren Kolbenstange definiert wird.

In einem weiteren Aspekt wird beschrieben, dass das innere Volumen der Kavitätals Spalt zwischen einer äusseren Mantelfläche der inneren Kolbenstange und einer inneren Mantelfläche der äusseren Kolbenstange definiert wird.

In einem weiteren Aspekt wird beschrieben, dass die Kavität am distalen Ende der inneren Kolbenstange lokalisiert ist und in etwa eine zylindrische Form aufweist.

In einem weiteren Aspekt wird beschrieben, dass das viskose Fluid eine Newton'sche Flüssigkeit ist.

In einem weiteren Aspekt wird beschrieben, dass das viskose Fluid eine scherverdünnende oder strukturviskose Flüssigkeit ist.

In einem weiteren Aspekt wird beschrieben, dass das viskose Fluid eine thixotrope oder teilthixotrope Flüssigkeit ist. wobei es sich bei der viskosen Flüssigkeit um eine hyaluronsäurehaltige Flüssigkeit handeln kann.

In einem weiteren Aspekt wird beschrieben, dass es sich bei der viskosen Flüssigkeit um eine flüssige Silikonformulierung handeln kann.

In einem weiteren Aspekt wird beschrieben, dass das viskose Fluid eine rheopexe oder dilatante Flüssigkeit sein kann, wobei die Flüssigkeit Partikel enthalten und/oder eine pastöse Konsistenz aufweisen kann.

In einem weiteren Aspekt wird beschrieben, dass die Ausschütteinrichtung weiter ein Halteelement mit mindestens einem flexiblen Arm mit einem freien Ende umfassen kann, an welchem die mindestens eine freizugebende Struktur fest angeordnet ist.

Beschrieben wird auch eine Ausschüttvorrichtung nach dem vorhergehenden Absatz, wobei die mindestens eine Freigabestruktur eine Vertiefung ist und pro Freigabestruktur mindestens eine freizugebende Struktur vorhanden ist, wobei die mindestens eine freizugebende Struktur als Eingriffselement ausgebildet ist.

Beschrieben wird auch eine Ausschüttvorrichtung nach dem vorhergehenden Absatz, wobei die Ausschüttvorrichtung weiter eine Druckfeder umfasst, welche eine Kraft auf das Haltelement in proximale Richtung ausübt, wobei das Halteelement ein proximales Ende umfasst und wobei die Freigabe des mindestens einen Eingriffselementes eine axiale Bewegung des Halteelementes freigibt und durch die wirkende Kraft der Druckfeder eine Bewegung des Halteelementes in axiale Richtung erfolgt.

Beschrieben wird auch eine Ausschüttvorrichtung nach dem vorhergehenden Absatz, wobei die Ausschütteinrichtung einen Endanschlag, insbesondere am Mechanikhalter, umfasst, welcher die axiale Bewegung des freigegebenen Halteelementes in axial Richtung begrenzt, so dass im Moment der Begrenzung der Bewegung ein hörbares oder fühlbares Signal erzeugt wird.

Der erfindungsgemässe Autoinjektor weist ein Gehäuse und ein in dem Gehäuse angeordneten Produktbehälter auf. Bei dem Produktbehälter kann es sich insbesondere um eine Spritze oder einer Karpule handeln, welche einen Behälterkörper aufweist, an dessen distalem Ende eine Injektionsnadel entweder fest angeordnet ist oder eine Injektionsnadel angebracht werden kann. Der Behälterkörper umgibt einen Kolben, der in Bezug auf den Spritzenkörper verschiebbar ist und für die Produktausschüttung zu dem distalen Ende hin verschoben wird, wodurch das zwischen dem Kolben und der Injektionsnadel angeordnete flüssige Produkt, insbesondere ein hochvisköses Medikament, durch die Injektionsnadel aus dem Produktbehälter ausgeschüttet wird. Der Spritzenkörper kann an seinem proximalen, d. h. hinteren oder der Injektionsnadel entgegengesetzten Ende einen Flansch, der auch als Fingerflansch bezeichnet werden kann, aufweisen. Eine derartig ausgebildete Spritze ist als Standardspritze erhältlich, so dass für den Autoinjektor nicht zwingend eine speziell angepasste Spritze entwickelt werden braucht. Der Kolben liegt dicht an dem Innendurchmesser des Spritzenkörpers an.

Das Gehäuse ist vorzugsweise länglich und bildet die Längsachse des Autoinjektors. Das Gehäuse ist vorzugsweise hülsenförmig und oder zylindrisch. Beispielsweise kann der Behälter verschiebbar in dem Gehäuse angeordnet sein, d. h. für ein automatisches Einstechen relativ zu dem Gehäuse in distale Richtung verschiebbar sein, so dass die Nadelspitze aus einer Öffnung am distalen Ende des Autoinjektors hervortritt und automatisch in den Patienten eingestochen werden kann. Optional kann bei einer solchen Vorrichtung die Nadelspitze nach erfolgter Produktausschüttung in das distale Ende der Vorrichtung hinein bewegt werden, insbesondere der Produktbehälter relativ zu dem Gehäuse in die proximale Richtung bewegt werden.

In bevorzugten Bauformen des Autoinjektors ist der Produktbehälter entlang der Längsachse unverschiebbar in dem Gehäuse aufgenommen, insbesondere mittels eines Produktbehälterhalters oder Spritzenhalters, der den Produktbehälter axialfest hält und axialfest mit dem Gehäuse verbunden, insbesondere verrastet ist. Bevorzugt steht die Nadelspitze über das distale Ende des Gehäuses in distale Richtung vor. Hierdurch kann die Nadel mittels einer Bewegung des Gehäuses zum Patienten hin in die Einstichstelle eingestochen werden. Vorzugsweise ist eine Nadelschutzhülse vorgesehen, welche das distale Ende des Autoinjektors bildet und eine Öffnung für die Injektionsnadel aufweist, wobei die Nadel durch die Öffnung hindurchtreten kann. Die Nadelschutzhülse kann in ihrer Ausgangsposition in Bezug auf die Nadelspitze so angeordnet sein, dass die Nadelschutzhülse distal über die Nadelspitze steht oder dass die Nadelspitze distal über das distale Ende der Nadelschutzhülse steht. Die Nadelschutzhülse ist relativ zu dem Gehäuse aus ihrer Ausgangsposition in die proximale Richtung um einen Betätigungshub in eine betätigte Position verschiebbar, insbesondere in das Gehäuse verschiebbar, so dass die Nadel aus dem distalen Ende bzw. durch die Öffnung der Nadelschutzhülse hervortritt oder weiter hervortritt. Bevorzugt kann die Nadelschutzhülse um einen Nadelschutzhub aus der betätigten Position relativ zum Gehäuse in die distale Richtung in eine Nadelschutzposition verschoben werden, in der das distale Ende der Nadelschutzhülse distal über die Nadelspitze hinausragt, um eine potentielle Verletzungsgefahr nach erfolgter Verwendung der Vorrichtung bzw. nach erfolgter Produktausschüttung, die von einer freiliegenden Nadelspitze ausgehen würde, zu verringern. Die Nadelschutzhülse kann z. B. gegen die Kraft einer Feder, die als Nadelschutzfeder bezeichnet werden kann, in die proximale Richtung verschoben werden, wobei die Feder, die z. B. die weiter unten beschriebene zweite Feder oder eine hiervon separate Feder ist, welche die Nadelschutzhülse aus der betätigten Position in die distale Richtung, d. h. in die Nadelschutzposition verschieben kann. Der Autoinjektor kann ein z. B. federnd angeordnetes Verriegelungsglied aufweisen, welches die Nadelschutzhülse in ihrer Nadelschutzposition insbesondere in Bezug auf das Gehäuse verriegelt und ein Zurückschieben der Nadelschutzhülse in die proximale Richtung oder in das Gehäuse blockiert. Das Verriegelungsglied verriegelt die Nadelschutzhülse zumindest so, dass die Nadel nicht aus dem distalen Ende der Nadelschutzhülse hervortreten kann. Die Nadelschutzhülse kann z. B. aus der Nadelschutzposition nur soweit in die proximale Richtung verschoben werden, dass die Nadelspitze nicht aus dem distalen Ende der Nadelschutzhülse hervortritt.

Der Autoinjektor umfasst ferner ein Vortriebsglied, das zumindest während der Produktausschüttung auf den Kolben wirkt, insbesondere an dem Kolben anliegt, und eine erste Feder, die direkt oder bevorzugt indirekt auf das Vortriebsglied wirkt. Das Vortriebsglied kann z. B. hülsenförmig sein. Die Feder kann vor dem Gebrauch gespannt werden z.B. als ein Zwischenschritt oder während einer Dosiseinstellung. Bevorzugt kann die Feder bereits bei Auslieferung des Autoinjektors mit so viel Energie vorgespannt sein, dass die Energie für mehrere Ausschüttungen der Produktdosis ausreicht, insbesondere für die Ausschüttung des gesamten aus dem Produktbehälter ausschüttbaren Produkts ausreicht.

Das Vortriebsglied kann mehrteilig, vorzugsweise zweiteilig ausgebildet sein. Dabei kann das Vortriebsglied aus einem äusseren Vortriebsglied und einem inneren Vortriebsglied bestehen, wobei das äussere Vortriebsglied auf den Kolben wirkt und das innere Vortriebsglied auf das äussere Vortriebsglied wirken kann. In einer erfindungsgemässen Ausführung sind äusseres und inneres Vortriebsglied länglich und hülsenförmig ausgestaltet, wobei das innere Vortriebsglied zumindest teilweise im äusseren Vortriebsglied gelagert und angeordnet ist. Grundsätzlich können inneres und äusseres Vortriebsglied für sich genommen relativ zueinander bewegbar, insbesonder drehbar ausgestaltet sein.

Der Autoinjektor kann ferner ein Rotationsglied umfassen, dessen Drehung bewirkt, dass die Federenergie an das Vortriebsglied abgegeben wird, wodurch das Vortriebsglied in distale Richtung bewegt wird. Bei einer mehrteiligen Ausgestaltung des Vortriebsglieds wird die Federenergie vorzugsweise vom Rotationsglied auf das innere Vortriebsglied übertragen, welches seinerseits den Vortrieb an das äussere Vortriebsglied überträgt, wobei die Übertragung des Vortriebs von innerem auf äusseres Vortriebsglied vorteilhaft nicht permanent ist, sondern abhängig vom Ausschüttzustand ist.

Das Rotationsglied kann mit der Feder, insbesondere einer Torsionsfeder oder Drehfeder, verbunden sein, die die zur Produktausschüttung notwendige Energie speichert und bei Bedarf abgibt. Grundsätzlich kann die Feder wendelförmig oder vorzugsweise spiralförmig sein. Die Feder kann aus einem Draht oder vorzugsweise aus einem bandförmigen Material, insbesondere Federstahl, gewickelt sein. Spiralförmig gewickelte Federn werden auch als Uhrenfedern oder Triebfedern bezeichnet.

Das Drehmoment der Feder muss zwischen dem Rotationsglied und dem u. U. mehrteiligen Vortriebsglied wirken. Die Feder kann mit dem Rotationsglied oder direkt mit dem Vortriebsglied und /oder mit einem beliebigen Element, welches axial und / oder rotativ fest in dem Gehäuse angebracht ist verbunden sein, oder mit einem Element welches axial und / oder rotativ gegenüber Gehäuse verschiebbar ist, verbunden sein. Die Feder kann axial fest in dem Gehäuse angebracht sein, sie kann aber auch axial verschiebbar zum Gehäuse sein.

Z.B. kann das Rotationsglied mit einem Ende an die Feder gekoppelt sein, wobei das andere Ende der Feder mit einem anderen Element, welches gegenüber dem Gehäuse insbesondere axial oder rotativ beweglich ist, verbunden sein kann. In einer bevorzugten Ausführungsform ist das andere Ende der Feder mit dem Gehäuse oder einem gehäusefestem Element verbunden.

Das Rotationsglied und / oder das Vortriebsglied und /oder ein anderes Element, welches mit dem Vortriebsglied gekoppelt ist, kann ein Gewinde mit einer variablen Steigung aufweisen, wobei in einem ersten Bereich das Gewinde eine grosse Steigung aufweisen kann und in weiteren Bereichen unterschiedlich grosse Steigungen möglich sind.

Aus Toleranzgründen kann im Auslieferungszustand des Autoinjektors ein Abstand zwischen Kolbenstange und Kolben bestehen. Bei der Herstellung wird versucht, den Abstand so klein wie möglich zu halten, damit der Aufprall der Kolbenstange auf den Kolben nicht zu einem Glasbruch führt. Dieser Abstand zwischen Kolbenstange und Kolben wird auch Beschleunigungsweg genannt. Um die Beschleunigung der Kolbenstange im Beschleunigungsweg zu kontrollieren bzw. abzubremsen, und Glasbruchrisiko zu minimieren, wird für den Anfang der Kolbenstangenbewegung ein Gewindeeinlaufweg mit einer grossen Steigung insbesondere auf dem Rotationsglied und / oder Vortriebsglied gewählt. Bevorzugt ist der Axialabschnitt des Gewindeeinlaufwegs grösser als der Beschleunigungsweg. Des Weiteren können in einer Lagerposition die Axialkräfte, welche insbesondere durch die Gewindeübersetzung aus dem Drehmoment der Feder entstehen durch eine grosse Steigung gering gehalten werden.

Das Gewinde bzw. die Gewindesteigung kann über die Länge des Rotationsglieds und / oder des Vortriebsglieds und / oder des Gehäuses variieren. Das Gewinde kann ein- oder mehrgängig sein. Vorzugsweise ist das Gewinde zweigängig. Die Steigung kann progressiv oder degressiv sein. Z.B. kann ein weiterer Bereich des Rotationsglieds eine kleinere Steigung aufweisen als der erste Bereich, wobei die grösste Gewindesteigung vorzugsweise nicht selbsthemmend sein kann.

Mit solch einem variierenden Gewinde ist es möglich, den Abfall des Federkraftmoments zu kompensieren und während der Ausschüttung die Ausschüttkraft in einem konstanten Bereich zu halten. Es ist möglich, dass am Ende der Ausschüttbewegung eine kleine Gewindesteigung gewählt wird und somit die Ausschüttkraft erhöht wird, damit z. B. eine Stopfenreibungskraft welche am Ende der Ausschüttung zunehmen kann, und eine vollständige Ausschüttung gewährleistet werden kann. Das Rotationsglied und / oder das Vortriebsglied und / oder das Gehäuse kann mehrere Bereiche mit verschiedenen Gewindesteigungen aufweisen. Z.B. kann das Gewinde eine grosse Gewindesteigung für den Gewindeeinlauf aufweisen und danach einen Bereich mit einer immer kleiner werdenden Gewindesteigung -für eine langsame Ausschüttung- und in einem Bereich mit einer kleinen Gewindesteigung -um ein vollständiges Ausschütten zu gewährleisten- enden. Selbstverständlich ist es auch möglich, dass die Gewindesteigung nach dem Gewindeeinlauf von einer kleinen Steigung in eine grosse Steigung verläuft, um am Anfang der Ausschüttung eine hohe Ausschüttkraft und am Ende der Ausschüttung eine kleine Ausschüttkraft zu erhalten.

Die Drehung des Rotationsglieds und / oder des Vortriebsglieds um einen bestimmten Drehwinkel bewirkt den axialen Vorschub des u. U. mehrteiligen Vortriebsglieds um einen entsprechenden Ausschütthub. Variable Steigungswinkel ergeben variablen axialen Vorschub des Vortriebsglieds bei gleichem Drehwinkel. Durch eine Freigabe des Vortriebsglieds kann der Feder erlaubt werden, dass sie das Vortriebsglied in distale Richtung bewegen kann. Insbesondere kann das Rotationsglied so mit dem Vortriebsglied gekoppelt sein, dass es bei der Auslösung bzw. Freigabe des Vortriebsglieds für eine Produktausschüttung für eine Drehung relativ zu dem Gehäuse freigegeben ist und im nicht ausgelösten Zustand des Vortriebsglieds für eine Rotation relativ zu dem Gehäuse gesperrt ist.

Vorteilhaft kann das Rotationsglied in einem Eingriff, insbesondere einem Gewindeeingriff mit dem Vortriebsglied sein, wobei der Gewindeeingriff bzw. die Gewindemutter am Vortriebsglied oder am Rotationsglied ein oder mehrere Gewindesegmente haben kann, vorzugsweise können es zwei Gewindesegmente sein. Bevorzugt ist das Gewindesegment so gewählt, dass ein Gewinde mit einer variablen Steigung hemmungsfrei durch die Gewindesegmente gedreht werden kann, Z. B. kann das Gewindesegment in einer abgewickelten Darstellung eine Kreisform oder eine ovale Form aufweisen, insbesondere kann der Umfang zu einer Längsachse hin, verschiedene Winkel aufweisen. Bei einer mehrteiligen Ausgestaltung des Vortriebsgliedes besteht der Gewindeengriff zwischen dem Rotationsglied und vorzugsweise dem inneren Vortriebsglied.

Bevorzugt kann eine Flanke des Gewindesegments des Vortriebsglieds oder des Rotationsglieds verschiedene Steigungswinkel aufweisen.

Vorzugsweise wird das Gewindesegment mit insbesondere Strichberührungen an dem Gewinde des Rotationsglieds geschraubt, wobei immer ein anderer Bereich des Gewindesegments das Gewinde berührt.

Insbesondere kann bei einer Schraubbewegung zwischen der Gewindestange oder dem Vortriebsglied und dem mindestens einem Gewindesegment des Vortriebsglieds bzw. der Gewindestange, die Flanke des mindestens einen Gewindesegments an dem Gewinde mit der variablen Steigung der Gewindestange oder des Vortriebsglieds geschraubt werden, wobei unterschiedliche Bereiche der Flanke das Gewinde mit der variablen Steigung berühren.

So ist bei der Schraubbewegung zwischen der Gewindestange und dem Vortriebsglied der Bereich der Gewindestange, welcher die grösste Gewindesteigung aufweist, mit dem Bereich des Gewindesegments, welcher den grössten Steigungswinkel aufweist in Berührung oder der Bereich der Gewindestange welcher die kleinste Gewindesteigung aufweist, mit dem Bereich des Gewindesegments welcher den kleinsten Steigungswinkel aufweist, in Berührung.

In einem alternativen Beispiel können das Rotationsglied eine Gewindemutter und das Vortriebsglied eine Gewindestange aufweisen oder sein, wobei das Gewinde der Gewindemutter in das Gewinde der Gewindestange eingreift und insbesondere nicht selbsthemmend ist.

Vorzugsweise ist das Rotationsglied axial fest in Bezug auf das Gehäuse oder kann sich zumindest in eine, vorzugsweise in distaler Richtung axialfest an dem Gehäuse oder einem gehäusefesten Element, wie z. B. dem Mechanikhalter abstützen, es ist auch möglich, dass das Rotationsglied einen Axialhub gegenüber dem Gehäuse ausführen kann. Insbesondere kann das Rotationsglied axial verschiebbar sein, wenn das Element an dem sich das Rotationsglied abstützt auch eine axiale Bewegung ausübt oder das Rotationsglied eine Gewindeverbindung zu einem gehäusefesten Element oder zu einem zum Gehäuse axial verschiebbaren Element aufweist.

Es ist auch möglich, dass mehrere Steuerkurven bzw. Gewinde zwischen verschiedenen Elementen wirken. Die verschiedenen Gewinde können ergänzend wirken, das heisst, für den axialen Weg addieren sich die Gewindesteigungen zueinander oder sie subtrahieren sich voneinander, somit erreicht man eine Übersetzung oder eine Untersetzung. Beispielsweise kann das Rotationsglied eine Gewindeverbindung zum Vortriebsglied aufweisen und das Vortriebsglied eine Gewindeverbindung zum Gehäuse oder zu einem gehäusefesten Element, wobei das Vortriebglied auch eine Rotationsbewegung ausführen kann. Es ist auch möglich, dass das Rotationsglied Gewindeverbindungen zum Vortriebsglied und zu einem gehäusefesten Element aufweist, wobei diese Gewindeabschnitte sich voneinander unterscheiden können und jeweils entweder variabel oder konstante Steigungsbereiche aufweisen können.

Die Gewinde können jeweils innen oder aussen an dem Rotationsglied und / oder an dem Vortriebsglied angebracht sein. Es können alle Gewindeverläufe an den verschiedenen Elementen variabel oder nur einer davon variabel sein. Z.B. kann das Rotationsglied ein stetig variables und / oder ein unstetig variables Gewinde und das Vortriebsglied eine stetige Gewindesteigung und / oder eine unstetig variables Gewindesteigung aufweisen.

Vorzugsweise kann das Gewinde mit der variablen Steigung, zumindest einen Bereich mit einer stetigen Steigungsvariation aufweisen oder/ und die Steigungsvariation des Gewindes mit der variablen Steigung zumindest bereichsweise unstetig verlaufen.

Bevorzugt können alle Elemente Gewindesegmente aufweisen und so gewählt sein, dass ein variables Gewinde hemmungsfrei durch die Gewindesegmente gedreht werden kann.

Die Gewinde, bzw. die Gewindesteigungen können variabel also progressiv oder degressiv verlaufen. Es ist auch möglich, dass die Gewinde sprunghafte Steigungen, also Bereiche mit unterschiedlicher Steigung aufweisen. Selbstverständlich kann das Gewinde mit der variablen Steigung, Bereiche mit einer stetigen Steigungsvariation aufweisen oder/ und die Variation des Gewindes mit der variablen Steigung verläuft zumindest bereichsweise unstetig.

In einer Ausführungsform ist das Vortriebsglied rotativ fest in Bezug auf das Gehäuse. Bei einer mehrteiligen Ausgestaltung des Vortriebsgliedes können in einer Variante alle Teile des Vortriebsgliedes gegenüber dem Gehäuse rotativ fest zum Gehäuse angeordnet sein. In einer weiteren Variante können einzelne oder mehrere Teile des Vortriebsgliedes in bestimmten Zuständen rotativ fest in Bezug auf das Gehäuse angeordnet sein. In einer bevorzugten, erfindungsgemässen Variante ist das Vortriebsglied zweiteilig ausgestaltet, bestehend aus einem inneren und einem äusseren Vortriebsglied, wobei beide Teile vor und während des eigentlich Ausschütthubs des Vortriebsgliedes gegenüber dem Gehäuse rotativ fest sind und die Verdrehsicherung zum Gehäuse des inneren Vortriebsglieds am Endes des Ausschütthubs gelöst wird.

Durch die Verschiebung des Vortriebsglieds um den Ausschütthub wird auch der Kolben verschoben. Sofern im Auslieferungszustand zwischen dem Kolben und dem Vortriebsglied ein Abstand besteht, ist der Ausschütthub des Kolbens kleiner als der Ausschütthub des Vortriebsglieds, was bevorzugt ist, da der Kolben so bis zur Verwendung unbelastet bleibt, wodurch eine ungewollte vorzeitige Produktausschüttung vermieden wird. Grundsätzlich ist es aber auch möglich, dass das Vortriebsglied im Auslieferungszustand und nicht erst bei der Produktausschüttung an dem Kolben anliegt. Sofern im Auslieferungszustand das Vortriebsglied an dem Kolben bereits anliegt, entspricht der Ausschütthub des Kolbens dem Ausschütthub des Vortriebsglieds.

Bei Varianten des Autoinjektors mit einer Nadelschutzhülse ist es bevorzugt, dass die Nadelschutzhülse auf die zweite Feder wirkt, wobei die Nadelschutzhülse für die Auslösung der Produktausschüttung aus ihrer Ausgangsposition relativ zu dem Gehäuse und entlang der Längsachse des Autoinjektors in die proximale Richtung, d. h. entgegen die Ausschüttrichtung verschiebbar ist, insbesondere um den Betätigungshub. Hierdurch wird die zweite Feder gespannt und vorzugsweise auch die Produktausschüttung, insbesondere die Bewegung des Vortriebsglieds in die Ausschüttrichtung, freigegeben bzw. ausgelöst. Die Nadelschutzhülse wird vorzugsweise dadurch aus ihrer Ausgangsposition um den Betätigungshub in ihre betätigte Position bewegt, indem ihr distales Ende an die Einstichstelle des Patienten angedrückt wird, wobei das Gehäuse relativ zur Nadelschutzhülse in Richtung Einstichstelle verschoben wird, so dass die Nadelschutzhülse den Betätigungshub relativ zu dem Gehäuse ausführt. Hierbei wird auch die aus dem distalen Ende der Nadelschutzhülse hervortretende Nadel in die Einstichstelle eingestochen. Nach der erfolgten Produktausschüttung, insbesondere nach z. B. einer kurzen Wartezeit, wie z. B. 3 bis 10 Sekunden, wird der Autoinjektor von der Einstichstelle abgenommen, wodurch die Nadelschutzhülse relativ zu dem Gehäuse aus ihrer betätigten Position um den Nadelschutzhub in die Nadelschutzposition verschoben wird, insbesondere mittels der in der zweiten Feder gespeicherten Federenergie. Der Beginn der Wartezeit, als Endsignal, oder das Ende der Wartezeit, als verzögertes Endsignal, wird durch ein akustisches oder taktiles Signal der benutzenden Person zur Kenntnis gebracht. Durch das Abnehmen des Autoinjektors von der Einstichstelle wird auch die Nadel aus der Einstichstelle gezogen.

In bestimmten Ausführungsformen kann kinematisch zwischen der zweiten Feder und der Nadelschutzhülse ein Schaltmodul angeordnet sein, wobei das Schaltmodul von der Nadelschutzhülse in die proximale Richtung mitgenommen wird, wenn die Nadelschutzhülse aus ihrer Ausgangsposition in die proximale Richtung oder in die betätigte Position verschoben wird, und die Nadelschutzhülse in die distale Richtung verschiebt, wenn die auf das Schaltmodul wirkende Feder das Schaltmodul in die distale Richtung verschiebt. Das Schaltmodul oder ein Teil davon, wie z. B. eine Schalthülse, kann mit der Nadelschutzhülse einteilig sein oder z. B. formschlüssig verbunden, wie z. B. verschnappt, sein oder lose an der Nadelschutzhülse anliegen. Das Schaltmodul kann ein einziges Teil sein oder mehrere Teile umfassen, wobei ein mehrteiliges Schaltmodul zumindest die Schalthülse und eine Sperrhülse aufweisen kann. Die Sperrhülse kann relativ zu der Nadelschutzhülse und/oder Schalthülse z. B. entlang der Längsachse verschiebbar sein. Beispielsweise können sich die Feder an der Schalthülse und die Schalthülse an der Nadelschutzhülse abstützen. Zwischen der Sperrhülse und der Schalthülse kann ein z. B. unidirektional wirkendes Verriegelungsglied vorgesehen sein, das z. B. von der Sperrhülse gebildet wird und in die Schalthülse, insbesondere in eine Ausnehmung oder in das distale Ende, eingreift. Das Verriegelungsglied ist vorzugsweise so ausgestaltet, dass die Schalthülse während ihrer Bewegung relativ zu dem Gehäuse in die proximale Richtung die Sperrhülse über das Verriegelungsglied mitnimmt, insbesondere während der Bewegung der Nadelschutzhülse aus ihrer Ausgangsposition in die betätigte Position, und während ihrer Bewegung relativ zu dem Gehäuse in die proximale Richtung relativ zu der Sperrhülse in eine Sperrposition verschoben wird, insbesondere während der Verschiebung der Nadelschutzhülse aus ihrer betätigten Position in die Nadelschutzposition, wobei in der Sperrposition das oder ein anderes Verriegelungsglied, wie z. B. das weiter oben genannte, eine Bewegung der Schalthülse relativ zu der Sperrhülse in die proximale Richtung sperrt. Hierdurch wird vorteilhaft verhindert, dass die Nadelschutzhülse aus ihrer Nadelschutzposition für eine erneute Freigabe der Nadelspitze in das Gehäuse zurückgeschoben werden kann.

Beispielsweise kann die Schalthülse eine erste Ausnehmung aufweisen, in welche das Verriegelungsglied der Sperrhülse lösbar eingreift, wenn die Nadelschutzhülse aus ihrer Ausgangsposition in ihre betätigte Position verschoben wird. Z. B. kann die Schalthülse eine zweite Ausnehmung aufweisen, in welche das Verriegelungsglied oder ggf. das andere Verriegelungsglied eingreift, wenn die Nadelschutzhülse in ihrer Nadelschutzposition ist. Die erste und zweite Ausnehmung können vorzugsweise mit einem Abstand, der in etwa dem Nadelschutzhub entspricht, zueinander entlang der Längsachse angeordnet sein. Selbstverständlich ist auch eine Umkehr der Anordnung der Ausnehmungen und des Verriegelungsglieds oder der Verriegelungsglieder möglich, d. h., dass das zumindest eine Verriegelungsglied an der Schalthülse und die mindestens eine Ausnehmung, d. h. die erste Ausnehmung und ggf. die zweite Ausnehmung an der Sperrhülse gebildet sein können.

Das Verriegelungsglied und ggf. das andere Verriegelungsglied können federnd, insbesondere jeweils an einem federnden Arm, angeordnet sein. Vorzugsweise kann die Schalthülse die Sperrhülse umgeben und/oder führen.

Weiterhin kann der Autoinjektor eine Verschlusskappe bzw. ein proximales Gehäuseteil aufweisen, welches am proximalen Ende des Gehäuses anbringbar ist und das proximale Ende des Autoinjektors bildet. Bevorzugt kann die Kappe oder das proximale Gehäuseteil Raum für die Antriebsfeder, bzw. erste Feder insbesondere Spiralfeder schaffen. Die Verschlusskappe oder das proximale Gehäuseteil kann mit dem Gehäuse so gekoppelt sein, dass sie sich gegenüber verdrehen lässt. Z.B. kann das proximale Gehäuseteil ein Dosierknopf oder ein Ladeknopf sein, wodurch die Feder vor der Injektion gespannt werden kann. In einer bevorzugten Ausführungsform kann die Verschlusskappe oder das proximale Gehäuseteil mit dem Gehäuse formschlüssig, alternativ kraft- oder stoffschlüssig verbunden sein. Besonders bevorzugt kann die Kappe oder das zweite Gehäuseteil lösbare und unlösbare Verbindungen aufweisen. Bevorzugt kann die Kappe oder das proximale Gehäuseteil in einem ersten Montageschritt lösbar mit dem Gehäuse verbunden werden und in einem zweiten Montageschritt zum Einfügen des Produktbehälters von dem Gehäuse gelöst und nach dem Einfügen des Spritzenkörpers unlösbar miteinander verbunden werden. Vorzugsweise wird die Kappe oder das proximale Gehäuseteil in dem zweiten Montageschritt unlösbar mit dem Gehäuse verrastet. Eine separate Kappe bzw. ein proximales Gehäuseteil mit lösbaren und unlösbaren Verrastungen hat den Vorteil, dass die Montage der Vorrichtung erleichtert wird, wobei für die Endmontage zumindest ein Teil der Bauteile über das proximale Ende des Gehäuses eingebaut werden und mit der Kappe in der Vormontage lösbar fixiert werden. Nach vorübergehender Entfernung der Kappe bzw. des proximalen Gehäuseteils in der Endmontage kann der Spritzenkörper über das proximale Ende des Gehäuses eingesetzt und die beiden Gehäuseteile unlösbar miteinander formschlüssig verrastet, alternativ kraft- oder stoffschlüssig zu verbunden werden.

In bevorzugten Ausführungen kann der Autoinjektor ein Halteelement aufweisen, an dem sich z. B. ein Ende der zweiten Feder, insbesondere das proximale Ende der zweiten Feder, abstützt. Alternativ kann sich die zweite Feder mit ihrem proximalen Ende an dem Gehäuse oder einem gehäusefesten Element abstützen.

Die zweite Feder kann sich mit ihrem distalen Ende z.B. an dem Gehäuse oder einem gehäusefesten Element abstützen. Besonders bevorzugt stützt sich die zweite Feder mit ihrem distalen Ende an der Nadelschutzhülse oder einem Element, welches, insbesondere bei der Verschiebung der Nadelschutzhülse relativ zu dem Gehäuse, mit der Nadelschutzhülse verschoben wird, ab. Z.B kann das Element das Schaltmodul oder insbesondere die Schalthülse sein. Das Halteelement selbst kann gehäusefest oder in Bezug auf das Gehäuse verschiebbar angeordnet sein. Das Halteelement kann ein erstes Eingriffselement aufweisen, welches vor dem Auslösen der Produktausschüttung in das Vortriebsglied eingreift, wodurch das Vortriebsglied daran gehindert wird, sich relativ zu dem Halteelement und/oder dem Gehäuse in die Ausschüttrichtung zu bewegen. Der Eingriff des ersten Eingriffselements in das Vortriebsglied ist für die Produktausschüttung lösbar. Wenn der Eingriff gelöst ist, ist das Vortriebsglied für die Bewegung in die Ausschüttrichtung freigegeben. Die erste Feder kann das Vortriebsglied relativ zu dem Halteelement und/oder dem Gehäuse um den Ausschütthub in die Ausschüttrichtung verschieben. Das Vortriebsglied kann eine Ausnehmung für das erste Eingriffselement des Halteelements aufweisen, wobei diese Kopplung zwischen dem Vortriebsglied und dem Halteelement gelöst ist, wenn das Halteelement, insbesondere das erste Eingriffselement, aus dem Eingriff mit dem Vortriebsglied, insbesondere der Ausnehmung des Vortriebsglieds ausgerückt ist. Bei einem mehrteilig ausgestalteten Vortriebsglied kann die Ausnehmung bevorzugt auf dem äusseren Vortriebsglied angeordnet sein. Insbesondere kann das erste Eingriffselement dadurch aus dem Eingriff mit dem Vortriebsglied gelöst werden, indem die Nadelschutzhülse aus der Ausgangsposition um den Betätigungshub in die betätigte Position verschoben wird. Beispielsweise kann das erste Eingriffselement von der Nadelschutzhülse oder dem Schaltmodul, insbesondere der Sperrhülse in den axialfesten Eingriff mit dem Vortriebsglied gehalten werden, wenn die Nadelschutzhülse nicht in ihrer betätigten Position oder in ihrer Ausgangsposition ist. Beispielsweise kann ein Innenumfang der Nadelschutzhülse oder des Schaltmoduls, insbesondere der Sperrhülse, das erste Eingriffselement in dem Eingriff mit dem Vortriebsglied halten.

Durch das Verschieben der Nadelschutzhülse in ihre betätigte Position kann die Nadelschutzhülse oder das Schaltmodul, insbesondere die Sperrhülse, dem ersten Eingriffselement erlauben, insbesondere mit einer Bewegung quer zu der Längsachse des Autoinjektors aus dem Eingriff mit dem Vortriebsglied auszurücken. Beispielsweise kann eine erste Ausnehmung, insbesondere für das zweite Eingriffselement, welche an der Nadelschutzhülse oder dem Schaltmodul, insbesondere der Sperrhülse, gebildet ist, bezogen auf die Längsachse auf der gleichen Position angeordnet sein, wie das erste und/oder zweite Eingriffselement, so dass das erste Eingriffselement aus dem Eingriff mit dem Vortriebsglied ausrücken kann. Beispielsweise kann das Vortriebsglied das erste Eingriffselement aus dem Eingriff mit dem Vortriebsglied drücken, wenn die Nadelschutzhülse in ihrer betätigten Position ist. Das erste Eingriffselement kann z. B. radial zu der Längsachse hinweisen und/oder an einem federnden Arm des Halteelements angeordnet sein.

Das Halteelement kann - wie erwähnt - ein zweites Eingriffselement aufweisen, welches durch die Ausrückbewegung des ersten Eingriffselements aus dem Vortriebsglied in einen Eingriff mit der Nadelschutzhülse oder dem Schaltmodul, insbesondere der Sperrhülse bewegbar ist. Das zweite Eingriffselement kann z. B. an dem Arm, an dem das erste Eingriffselement angeordnet ist, angeordnet sein und/oder z. B. radial von der Längsachse weg weisen. Das erste Eingriffselement und das zweite Eingriffselement können so aufeinander abgestimmt sein, dass das zweite Eingriffselement bereits axialfest in seine Ausnehmung, welche von der Nadelschutzhülse oder dem Schaltmodul, insbesondere der Sperrhülse, gebildet wird, eingreift, wenn das erste Eingriffselement noch nicht vollständig aus dem Eingriff mit dem Vortriebsglied gelöst ist. Hierdurch wird vorteilhaft erreicht, dass zuerst die axialfeste Verbindung zwischen dem Halteelement und der Nadelschutzhülse oder dem Schaltmodul hergestellt ist, bevor die axialfeste Verbindung zwischen dem Halteelement und dem Vortriebsglied gelöst wird.

Insbesondere wenn das zweite Eingriffselement in seiner Ausnehmung ist, kann sich das Vortriebsglied relativ zu dem Halteelement in distale Richtung bewegen, insbesondere aufgrund der in der vorgespannten ersten Feder gespeicherten Energie. Das Vortriebsglied kann das zweite Eingriffselement daran hindern, aus dem axialfesten Eingriff mit der Nadelschutzhülse oder dem Schaltmodul, insbesondere der Sperrhülse auszurücken, wenn sich das Vortriebsglied relativ zu dem Halteelement in die distale Richtung bewegt.

Da die axialfeste Kopplung zwischen dem Vortriebsglied und dem Halteelement nun aufgehoben ist, ist das Halteelement von der zweiten Feder relativ zum Gehäuse nach proximal bewegbar. Insbesondere wird dabei die Sperrhülse um einen Abstand zwischen der Sperrhülse und einem Startsignalanschlag bewegbar. Die zweite Feder kann das Halteelement und/ oder die Sperrhülse auf diesem Abstand beschleunigen, wodurch die Sperrhülse mit einer Geschwindigkeit auf den Startsignalanschlag auftrifft, so dass ein Startimpuls abgegeben wird, der ein akustisches (hörbares) und/ oder taktiles (fühlbares) Signal erzeugt.

Der Startsignalanschlag kann von dem Gehäuse oder einem zumindest axialfest, vorzugsweise auch drehfest mit dem Gehäuse verbundenem Element gebildet werden. Beispielsweise kann dieses Element der Mechanikhalter sein.

Bevorzugt ist der Signalanschlag entlang der Längsachse des Autoinjektors so angeordnet, dass er in einer Flucht mit der Sperrhülse angeordnet ist. Hierdurch wird erreicht, dass die Sperrhülse mit einer Bewegung des Halteelements entlang der Längsachse des Autoinjektors an dem Startsignalanschlag anschlägt.

Der Autoinjektor weist einen Endsignalanschlag auf. Wie bereits erwähnt kann die zweite Feder eine auf das Halteelement entgegen der Ausschüttrichtung oder in die proximale Richtung wirkende Federkraft ausüben. Insbesondere kann sich die zweite Feder z. B. mit ihrem proximalen Ende an dem Halteelement abstützen.

Die Feder kann somit eine Mehrfachfunktion erfüllen, da sie Kraft zum Verschieben der Nadelschutzhülse bzw. des Schaltmoduls aufbringt und Kraft auf das Halteelement, für ein Startsignal und für ein Endsignal, ausübt.

Wie bereits oben erwähnt kann das Vortriebsglied das zweite Eingriffsglied daran hindern, aus dem Eingriff mit der Nadelschutzhülse oder dem Schaltmodul auszurücken, wenn sich das Vortriebsglied relativ zu dem Halteelement in die distale Richtung bewegt. Das Vortriebsglied erlaubt dem zweiten Eingriffsglied nach dem Ende des Ausschütthubs, dass es aus dem Eingriff mit der Nadelschutzhülse oder dem Schaltmodul bzw. der Sperrhülse ausrückt. Wenn das zweite Eingriffsglied am Ende der Ausschüttung aus dem Eingriff mit der Nadelschutzhülse oder dem Schaltmodul bzw. der Sperrhülse ausgerückt ist, wird das Halteelement von der zweiten Feder entgegen die Ausschüttrichtung beschleunigt und schlägt an dem Endsignalanschlag an.

Wie weiter oben erwähnt kann das Endsignal unmittelbar anschliessend an den Ausschütthub erzeugt werden. Alternativ, erfindungsgemäss und bevorzugt kann das Endsignal nach dem Ausschütthub erst mit einer Verzögerung generiert werden. Wie im Folgenden beschrieben kann die Verzögerung vorteilhaft mit einem mehrteiligen, insbesondere zweiteiligen Vortriebsglied erzeugt werden. Wie oben schon erwähnt umfasst das zweiteilige Vortriebsglied ein äusseres Vortriebsglied, welches gegenüber dem Gehäuse verdrehgesichert ist. Weiter umfasst das zweiteilige Vortriebsglied ein inneres Vortriebsglied, welches vor und während dem Ausschütthub des Vortriebsgliedes gegenüber Gehäuse verdrehgesichert ist. Diese Verdrehsicherung wird am Ende des Ausschütthubes gelöst. Somit bewirkt das weiter wirkende Drehmoment der Feder, welches über das Rotationsglied auf das innere Vortriebsglied übertragen wird, eine Rotation des inneren Vortriebsgliedes relativ zum äusseren Vortriebsglied. Das zweite Eingriffsglied wird am Ende des Ausschütthubes durch das proximale Ende des inneren Vortriebsgliedes, welches über das proximale Ende des äusseren Ausschüttgliedes hinausragt, in Eingriff mit Nadelschutzhülse oder Schaltmodul bzw. Sperrhülse gehalten. Auf dem Umfang des proximalen Endes des inneren Vortriebsgliedes sind entsprechend der Anzahl erster Eingriffsglieder die gleichen Anzahl Freigabeelemente, insbesondere Ausnehmungen oder Vertiefungen angeordnet. Vor und während dem Ausschütthub sind diese Freigabeelemente rotativ bezogen auf die Längsachse des Autoinjektors so orientiert, dass die ersten Eingriffglieder rotativ bezogen auf die Längsachse nicht dieselbe Orientierung haben wie die Freigabeelemente, so dass ein Ausrücken des zweiten Eingriffgliedes (es können auch mehr als eines sein, insbesondere zwei) erst möglich wird wenn die Freigabeelement durch Rotation des inneren Vortriebselement auf die ersten Eingriffglieder ausgerichtet wurden, so dass sich diese radial nach innen bewegen können. Sind die Freigabeelemente einmal ausgerichtet, so rückt das zweite Eingriffsglied aus und das Halteelement wird durch die zweite Feder beschleunigt und bewirkt das Endsignal.

Wie erwähnt ist erfindungsgemäss das innere Vortriebsglied zumindest teilweise innerhalb des äusseren Vortriebsgliedes gelagert und angeordnet. Die förmliche Ausgestaltung der beiden Vortriebsglieder ist so, dass sie zusammen eine innere Kavität umschliessen oder definieren, wobei das Volumen der Kavität durch eine äussere Oberfläche des inneren Vortriebsglieds und eine innere Oberfläche des äusseren Vortriebsglieds eingegrenzt wird. Die Kavität kann im weiteren Sinne zylindrisch, ringförmig oder auch spaltförmig zwisohen den beiden hülsenförmigen Vortriebsgliedem ausgestaltet sein. Ist die innere Kavität mit einem viskosen Fluid befüllt, so bildet dieses Fluid bei einer Rotation des inneren Vortriebgliedes relativ zum äusseren Vortriebsglied einen fluiden Widerstand. Dieser Widerstand kann dazu verwendet werden, die Rotation des inneren Vortriebsgliedes nach dem Lösen der Verdrehsicherung zum Gehäuse zu bremsen. In Folge wird somit auch die Erzeugung des Endsignals im Sinne der Erfindung verzögert. Über die Eigenschaften des Fluides lässt sich die Dauer der Verzögerung auf die Eigenschaften des Medikamentes einstellen. So brauchen insbesondere zähflüssige Medikamente eine längere Verzögerung des Endsignals, um sicherstellen zu können, dass die ganze Medikamentenmenge im Körper des Patienten angekommen ist. Das Fluid ist idealerweise eine nicht-flüchtige Flüssigkeit. Das Fluid kann eine einfache Newton'sche Flüssigkeit sein, wobei die Verzögerung über die Viskosität der Flüssigkeit eingestellt werden kann. Nicht weniger interessant im Sinne der Erfindung sind jedoch nicht Newton'sche Flüssigkeiten, insbesondere solche mit thixotropen Eigenschaften. Mit thixotrop ist gemeint, dass eine Flüssigkeit unter Belastung zeitabhängig ihre Viskosität ändert, im Falle von thixotropen Flüssigkeiten reduziert sich die Viskosität zeitabhängig (das gegenteilige Verhalten wird als rheopex bezeichnet). Thixotrope Flüssigkeiten können im Ruhezustand sehr zäh sein, sich allenfalls ähnlich zu Feststoffen verhalten - im Rahmen der vorliegenden Erfindung kann das ein grosser Vorteil sein, da eine thixotrope Flüssigkeit im Ruheszustand dort bleibt, wo sie ist und nicht wegfliesst. Unter einer Scherlast, verringert sich Viskosität unter Umständen zeitabhängig erheblich. Dem Fachmann sind eine Vielzahl thixotroper Flüssigkeiten zugänglich, insbesondere kieselgelhaltige oder hyaluronsäurehaltige Flüssigkeiten. Eine gute Alternative zu thixotropen Flüssigkeiten stellen strukturviskose Flüssigkeiten dar, welche wie thixotrope Flüssigkeiten im Ruhezustand eine erhöhte Viskosität aufweisen. Anders als bei thixotropen Flüssigkeiten reduziert sich die Viskosität mit steigender Schergeschwindigkeit und nicht abhängig von der Zeit. Beispielhaft seien hier Polymerlösungen oder lonomere genannt, welche die gewünschten Eigenschaften haben können.

Alternativ zu Fluiden, bei welchen die Viskosität abhängig von Zeit resp. Schergeschwindigkeit abnimmt, könnten auch Fluide mit gegenteiligen Eigenschaften vorteilhaft eingesetzt werden, insbesondere rheopexe sowie dilatante Flüssigkeiten, wobei darauf geachtet werden muss, dass die Viskositätszunahme nicht zu extrem ausfällt und die Verzögerung des Endsignal noch kontrolliert ist. Bei dilatanten Fluiden sind insbesondere partikelhaltive Flüssigkeiten respektive pastöse Substanzen denkbar, insbesondere Zinkpasten.

Die Verzögerung kann erfindungsgemäss weiter durch die Oberflächengestaltung von innerem und äusserem Vortriebsglied im Bereich des mit Fluid befüllten Volumens der Kavität beeinflusst. So können ruderartige oder knethakenförmige Strukturen vorhanden sein, welche während der Rotation des inneren Vortriebsgliedes den Schergrad im Fluid erhöhen und damit die Bewegung weiter bremsen, in dem Energie von der Feder ins Fluid übertragen wird.

Der Endsignalanschlag kann von dem Gehäuse oder einem zumindest axialfest, vorzugsweise auch drehfest mit dem Gehäuse verbundenem Element gebildet werden. Beispielsweise kann dieses Element der Mechanikhalter sein.

Bevorzugt ist der Endsignalanschlag entlang der Längsachse des Autoinjektors so angeordnet, dass er in einer Flucht mit dem Halteelement angeordnet ist. Hierdurch wird erreicht, dass das Halteelement mit einer Bewegung entlang der Längsachse des Autoinjektors an dem Endsignalanschlag anschlägt.

Insbesondere in Ausgestaltung des Autoinjektors, in denen die distale Ausnehmung für das zweite Eingriffselement des Halteelements von der Nadelschutzhülse oder der Schalthülse insbesondere der Sperrhülse gebildet wird, ist es bevorzugt, dass das zweite Eingriffselement am Ende des Ausschütthubs aus seiner Ausnehmung ausrückt, um die Nadelschutzhülse nach der Verabreichung des Produkts aus der betätigten Position in die Nadelschutzposition bewegen zu können. Das Vortriebsglied kann hierzu eine proximale Ausnehmung aufweisen, in welche das erste Eingriffselement einrücken kann, wobei das zweite Eingriffselement gleichzeitig aus seiner Ausnehmung ausrückt.

Die distale Ausnehmungen an der Sperrhülse kann auch das distale Ende der Sperrhülse bilden. Es ist auch möglich, dass die proximale Ausnehmung an dem Vortriebsglied das proximale Ende des Vortriebsglieds darstellen kann.

In Ausführungsformen, mit einem Schaltmodul, welches eine Schalthülse und eine Sperrhülse aufweist, ist es bevorzugt, dass die Sperrhülse mit einem nach innen weisenden Sperrarm in das Gehäuse oder in ein gehäusefesten Element, wie z. B. in den Mechanikhalter eingreift und so die Sperrhülse daran hindert, relativ zu dem Gehäuse in die distale Richtung bewegt zu werden, wobei die Schalthülse und/oder die Nadelschutzhülse relativ zu der Sperrhülse, insbesondere aufgrund der in der zweiten Feder gespeicherten Energie in die distale Richtung verschiebbar sind, wodurch die Nadelschutzhülse insbesondere in ihre Nadelschutzposition bewegt wird. Wie bereits beschrieben und nur der Vollständigkeit halber angemerkt, kann das Verriegelungsglied zwischen der Sperrhülse und der Schalthülse in einen Eingriff gelangen, der verhindert, dass die Schalthülse relativ zu der Sperrhülse in die proximale Richtung verschiebbar ist. Vorzugsweise wird eine Bewegung der Sperrhülse in die proximale Richtung dadurch verhindert, dass die Sperrhülse entweder an dem Gehäuse oder einem gehäusefesten Element, wie z. B. an dem Mechanikhalter, anstösst.

Weiterhin weist der Autoinjektor einen Produktbehälterhatter, insbesondere einen Spritzenhalter insbesondere für einen Autoinjektor, bei dem der Produktbehälter in Bezug auf das Gehäuse unverschiebbar ist bzw. für einen Autoinjektor der oben beschriebenen Art.

Die Erfindung geht von einem Spritzenmodul aus, welches insbesondere zur Verwendung in einem Autoinjektor vorgesehen ist. Insbesondere kann ein Autoinjektor vorgesehen sein, der ein solches Spritzenmodul aufweist. Das Spritzenmodul umfasst eine Spritze und einen Spritzenhalter. Die Spritze weist einen Spritzenkörper, einen Kolben und eine Nadel auf, wobei die Nadel z. B. unlösbar an einem Nadelhalteabschnitt der Spritze befestigt ist und der Kolben in einem zylindrischen Abschnitt des Spritzenkörpers verschiebbar angeordnet ist, wobei der Spritzenkörper einen sich verjüngenden Abschnitt oder Bereich aufweist, der zwischen dem zylindrischen Abschnitt und dem Nadelhalteabschnitt angeordnet ist. Die Spritze weist ferner eine Nadelschutzkappe auf, welche z. B. ein sogenanntes soft needle shield oder vorzugsweise ein rigid needle shield RNS sein kann. Ein soft needle shield ist vorzugsweise aus einem gummielastischen Kunststoff gebildet, wobei ein rigid needle shield aus einer Hülse aus Hartkunststoff gebildet ist, in der eine Hülse aus einem gummielastischen Kunststoff angeordnet ist. Die Hülse aus gummielastischem Kunststoff und die Hülse aus Hartkunststoff bilden zusammen das rigid needle shield. Die Nadelschutzkappe, welche die Nadel abdeckt und an dem sich insbesondere konisch in Richtung Nadelspitze erstreckenden Nadelhalteabschnitt befestigt ist, hält die Nadel vorzugsweise geschützt vor Schmutz und steril. Über dem sich verjüngenden Abschnitt zwischen dem zylindrischen Abschnitt und der Nadelschutzkappe, insbesondere der Hülse aus Hartkunststoff, ist eine Lücke gebildet.

Der Spritzenhalter weist mindestens ein Eingriffsglied, insbesondere eine Schulter, an welcher sich der verjüngende Abschnitt der Spritze in die distale Richtung abstützt und welche in die Lücke zwischen der Nadelschutzkappe und dem zylindrischen Abschnitt eingreift, auf. Vorteilhaft wird durch das Anliegen des sich verjüngenden Abschnitts an der mindestens einen Schulter verhindert, dass sich die Spritze relativ zu dem Spritzenhalter in die distale Richtung bewegen kann.

Es ist bevorzugt, dass zwischen der Schulter und der Nadelschutzkappe ein Spalt ist oder besteht, so dass die Nadelschutzkappe von der Schulter unbelastet bleibt. Hierdurch wird vorteilhaft verhindert, dass die Sterilität der Nadel durch ein unbeabsichtigtes Verschieben der Nadelschutzkappe durch die Schulter beeinträchtigt wird.

In bevorzugten Ausführungen kann der Spritzenkörper an seinem proximalen Ende einen Fingerflansch aufweisen, wobei zwischen dem Fingerflansch und dem Spritzenkörper ein Spalt gebildet ist, wenn der sich verjüngende Abschnitt an der Schulter anliegt, wodurch der Fingerflansch im Wesentlichen unbelastet bleibt. Hierdurch wird vorteilhaft verhindert, dass der Fingerflansch überbelastet wird und den Spritzenkörper zerbricht.

Es ist ferner bevorzugt, dass der Spritzenhalter mindestens ein Halteglied, insbesondere eine nach aussen gerichtete Abragung aufweist, mit dem der Spritzenhalter axialfest mit einem Gehäuse des Autoinjektors verbindbar oder verbunden ist, insbesondere verschnappt oder verschnappbar ist.

In bevorzugten Ausführungen kann der Spritzenhalter mindestens einen Nocken aufweisen, der federnd, insbesondere an einem Arm angeordnet ist und z. B. distal des Halteglieds angeordnet ist. Der mindestens eine Nocken kann eine Nadelschutzhülse an der Bewegung aus ihrer Ausgangsposition in ihre betätigte Position so hemmen oder hindern, dass beim Überschreiten einer auf die Nadelschutzhülse entlang der Längsachse L des Autoinjektors ausgeübten Grenzkraft der mindestens eine Nocken aus dem Eingriff mit der Nadelschutzhülse gedrückt wird, wodurch die Nadelschutzhülse schlagartig in ihre betätigte Position relativ zu dem Gehäuse verschiebbar ist.

Das Gehäuse des Autoinjektors kann z. B. einen Halteabschnitt aufweisen, der an dem Spritzenhalter, insbesondere an einer Aussenfläche oder einem Aussenumfang des Spritzenhalters anliegt und das mindestens eine Eingriffsglied daran hindert, sich quer zur Längsachse von der Längsachse weg zu bewegen. Insbesondere kann der Halteabschnitt ringförmig sein und das mindestens eine Eingriffsglied, vorzugsweise zwei oder drei oder vier Eingriffsglieder umgeben, so dass das mindestens eine Eingriffsglied innerhalb des Halteabschnitts angeordnet ist. Für die Montage bzw. das Einlegen der Spritze in den Spritzenhalter befindet sich der Spritzenhalter ausserhalb des Eingriffs mit dem Halteabschnitt des Gehäuses. Wenn die Spritze vollständig in den Spritzenhalter eingelegt ist, insbesondere das mindestens eine Eingriffsglied in die Lücke zwischen dem sich verjüngenden Abschnitt und der Nadelschutzkappe eingreift, wird das Spritzenmodul bzw. der Spritzenhalter in den Eingriff mit dem Halteabschnitt gebracht, so dass verhindert wird, dass sich das mindestens eine Eingriffsglied aus dem Eingriff mit dem verjüngenden Abschnitt quer zur Längsachse, insbesondere von der Längsachse weg oder nach aussen, bewegt.
Figur 1 eine Explosionsdarstellung des Basis-Autoinjektors 0,
Figuren 2a-2b der Autoinjektor 0 aus Figur 1 in einem Auslieferungszustand, wobei die Figuren 2a bis 2b durch die Längsachse der Vorrichtung verlaufende Schnittansichten sind, wobei die Schnittansichten um die Längsachse winkelversetzt sind,
Figuren 3a-3b die Vorrichtung und die Ansichten aus den Figuren 2a-2b, wobei sich eine Nadelschutzhülse in ihrer betätigten Position befindet,
Figuren 4a-4b die Vorrichtung und die Ansichten aus den Figuren 2a-2b, wobei ein Signal, welches den Anfang der Produktausschüttung signalisiert, erzeugt wird,
Figuren 5a-5b die Vorrichtung und die Ansichten aus den Figuren 2a-2b, wobei ein Vortriebsglied am Ende seines Ausschütthubs gezeigt wird,
Figuren 6a-6b die Vorrichtung und die Ansichten aus den Figuren 2a-2b, wobei ein Signal, welches das Ende der Produktausschüttung signalisiert, erzeugt wird,
Figuren 7a-7b die Vorrichtung und die Ansichten aus den Figuren 2a-2b, wobei die Nadelschutzhülse in ihrer Nadelschutzposition ist,
Figuren 8a-8c Darstellung des Rotationsglieds und des Vortriebsglieds des Basis-Autoinjektors und Varianten davon,
Figur 9 eine Explosionsdarstellung eines erfindungsgemässen Autoinjektors 100 in einer bevorzugten Ausführung,
Figuren 10a-10b der Autoinjektor 100 aus Figur 9 in einem Auslieferungszustand, wobei die Figuren 10a bis 10b durch die Längsachse der Vorrichtung verlaufende Schnittansichten sind, wobei die Schnittansichten um die Längsachse um 90° winkelversetzt sind,
Figuren 11a-11b die Vorrichtung und die Ansichten aus den Figuren 10a-10b, wobei sich eine Nadelschutzhülse in ihrer betätigten Position befindet,
Figuren 12a-12b die Vorrichtung und die Ansichten aus den Figuren 10a-10b, wobei ein Signal, welches den Anfang der Produktausschüttung signalisiert, erzeugt wird,
Figuren 13a-13b die Vorrichtung und die Ansichten aus den Figuren 10a-10b, wobei das mehrteilige Vortriebsglied am Ende seines Ausschütthubs gezeigt wird,
Figuren 14a-14b die Vorrichtung und die Ansichten aus den Figuren 10a-10b, wobei das innere Teil des mehrteiligen Vortriebsgliedes (innere Kolbenstange) in gedrehtem Zustand gezeigt wird,
Figuren 15a-15b die Vorrichtung und die Ansichten aus den Figuren 10a-10b, wobei ein Signal, welches das Ende der Produktausschüttung signalisiert, erzeugt wird,
Figuren 16a-16b die Vorrichtung und die Ansichten aus den Figuren 10a-10b, wobei die Nadelschutzhülse in ihrer Nadelschutzposition ist,
Figur 17 erfindungsgemässe Ausführung des mehrteiligen Vortriebsglieds,
Figur 18 weitere erfingsgemässe Ausführung des mehrteiligen Vortriebsglieds,
Figur 19 erfindungsgemässe Ausführung des Mechanikhalters.

Im Folgenden wird ein Basis-Autoinjektor beschrieben, welcher als Basis für die vorliegende Erfindung dient. Danach wird die Erfindung in diesen Basis-Autonjektor integriert und beschrieben. Dieses schrittweise Vorgehen soll es dem Fachmann ermöglichen, die Materie der Erfindung effizient und vollständig zu erfassen.

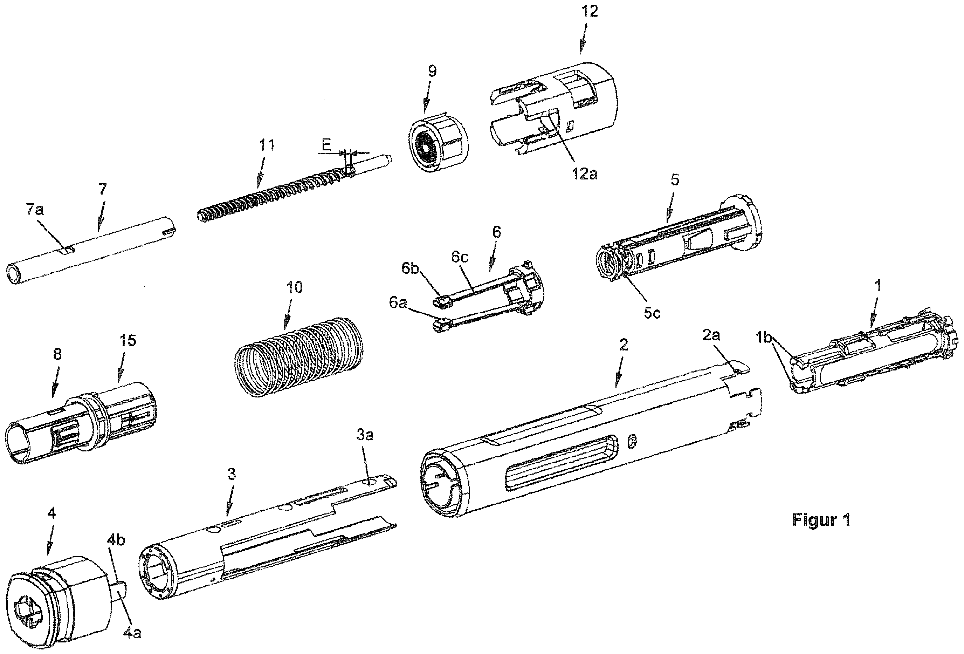

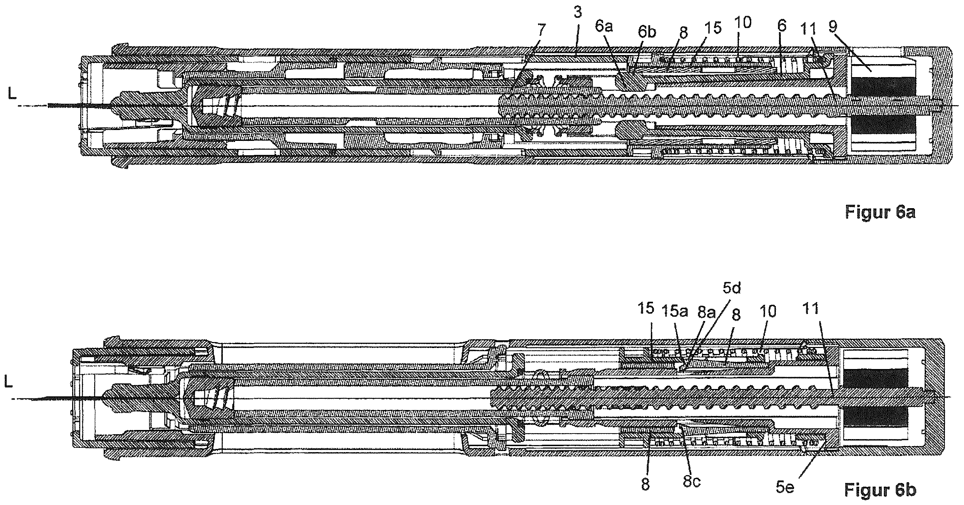

Bezugnehmend auf die Figuren 1-8c werden nun die strukturellen Merkmale und die Funktion des Basis-Autoinjektors beschrieben.

Der Autoinjektor 0 weist ein hülsenförmiges, längliches Gehäuse 2 mit einer Längsachse L auf, welches an seinem proximalen Ende eine Verschlusskappe 12 aufweist, die formschlüssig mit dem Gehäuse 2 dreh- und axialfest verbunden ist und das proximale Ende des Autoinjektors bildet. Die Verschlusskappe 12 ist mit dem Gehäuse 2 verschnappt. Hierfür weist die Verschlusskappe 12 ein Rastglied 12a auf, welches in eine Ausnehmung 2a am Gehäuse 2 eingerastet ist, vorzugsweise so, dass die Verschlusskappe 12 nicht oder nicht ohne weiteres von dem Gehäuse 2 lösbar ist.

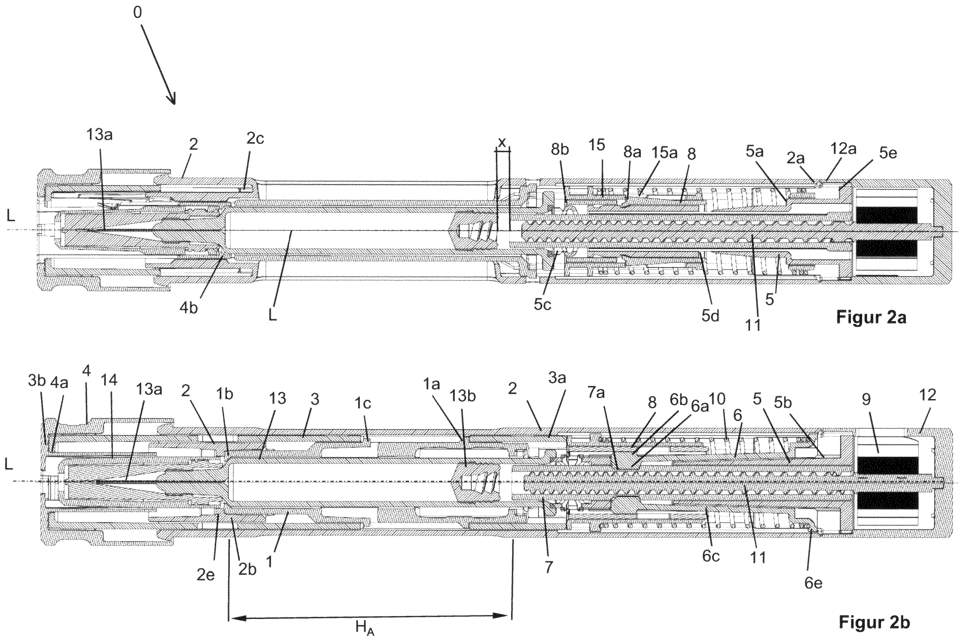

Am distalen Ende des Autoinjektors ist in seinem Auslieferungszustand (Figuren 2a-2b) eine Abziehkappe 4 angeordnet, die vor der Verwendung des Autoinjektors abgezogen, oder abgedreht, und entfernt wird.

Im Gehäuse 2 ist ein Produktbehälter 13 in der Gestalt einer Spritze in Bezug auf das Gehäuse 2 - abgesehen von der Montage des Autoinjektors - entlang der Längsachse L unverschiebbar aufgenommen. Der Produktbehälter 13 weist einen hülsenförmigen Spritzenkörper auf, der einen Kolben 13b, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel 13a auf, deren distales Ende von der Nadelspitze gebildet wird. Zwischen der Injektionsnadel 13a und dem Kolben 13b ist ein flüssiges Produkt, insbesondere Medikament, innerhalb des Spritzenkörpers angeordnet, wobei das flüssige Produkt durch Verschieben des Kolbens 13b in eine Ausschüttrichtung, d. h. in distale Richtung oder zu der Injektionsnadel 13a hin durch die hohle Injektionsnadel 13a aus dem Produktbehälter 13 ausgeschüttet wird. Der Spritzenkörper weist an seinem proximalen Ende einen sogenannten Fingerflansch auf, der radial nach aussen über den Aussenumfang des zylindrischen Spritzenkörpers ragt.

Der Produktbehälter 13 ist in einem Produktbehälterhalter, der als Spritzenhalter 1 bezeichnet wird, so aufgenommen, dass er zumindest gegen eine Bewegung entlang der Längsachse L in distale Richtung relativ zu dem Spritzenhalter 1 gesichert ist. Der Spritzenhalter 1 ist, wie am besten aus Figur 2a ersichtlich ist, mit dem Gehäuse 2 formschlüssig verbunden, insbesondere verrastet. Das Gehäuse 2 weist hierzu Ausnehmungen auf, in die Rastelemente, die hier am proximalen Ende des Spritzenhalters 1 gebildet werden, eingreifen. Der Spritzenhalter 1 weist mindestens eine nach innen ragende Schulter 1b auf, an der sich ein verjüngender Abschnitt des Produktbehälters 13, der distal des zylindrischen den Kolben 13b führenden Spritzenkörperabschnitts, angebracht ist, abstützt.

Um zu verhindern, dass der Produktbehälter 13 relativ zum Spritzenhalter 1 in die proximale Richtung verschiebbar ist, wird der Produktbehälter 13 an seinem proximalen Ende durch einen auf den Spritzenkörper wirkenden Halter in Eingriff mit der Schulter 1b gedrückt. Der Halter wird von einem Haltefederabschnitt 5c eines Mechanikhalters 5 gebildet. Der Mechanikhalter 5 ist in Bezug auf das Gehäuse 2 entlang der Längsachse L insbesondere unverschiebbar und/oder drehfest angeordnet. Der hülsenförmige Mechanikhalter 5 kann mit dem Gehäuse 2 verschnappt sein. Durch den Haltefederabschnitt 5c können Längenunterschiede des Produktbehälters 13, die aufgrund von Herstellungstoleranzen entstehen können, ausgeglichen werden, wobei der feste Sitz des Produktbehälters 13 an der Schulter 1b sichergestellt ist,

Der Produktbehälter 13 ist in Bezug auf das Gehäuse 2 so angeordnet, dass die Nadelspitze distal über das distale Ende des Gehäuses 2 übersteht. Im Ausgangs- oder Auslieferungszustand des Autoinjektors, d. h., wenn die Abziehkappe 4 an dem Autoinjektor angeordnet ist, wird die Nadel 13a von einer Nadelabdeckkappe 14, die in dem gezeigten Beispiel als sogenanntes und dem Fachmann bekanntes rigid needle shield, alternativ als soft needle shield, ausgebildet ist, abgedeckt, um die Nadel 13a vor Verschmutzung zu schützen bzw. die Nadel 13a und das Medikament steril zu halten. Das rigid needle shield 14 ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 1b ist zwischen dem Spritzenkörper und dem proximalen Ende des rigid needle shield 14 angeordnet, insbesondere so, dass zwischen dem rigid needle shield 14 und der Schulter 1b ein - wenngleich auch geringer - Spalt entsteht, um zu verhindern, dass die Schulter 1b eine Kraft auf das rigid needle shield 14 ausübt, wodurch z. B. die Sterilität der Nadel 13a oder des flüssigen Produkts gefährdet werden könnte. Die Abziehkappe 4 ist mit dem Gehäuse 2 oder einer Nadelschutzhülse 3 lösbar verschnappt, wobei diese Verschnappung gelöst wird, wenn die Abziehkappe 4 von dem Gehäuse 2 oder der Nadelschutzhülse 3 entfernt wird. Die Verschnappung wird in dem gezeigten Beispiel durch eine Schnappgeometrie 3b der Nadelschutzhülse 3 und einem Schnapphaken 4a der Abziehkappe 4 gebildet (Figur 2b). Diese Schnapphaken 4a sichern die Abziehkappe 4 weiter gegen eine proximale Bewegung relativ zum Gehäuse 2 indem sie auf dem Gehäuse 2 oder an einer distalen Stirnseite an dem Spritzenhalter 1 eine gehäusefeste Abstützung finden. Die Abziehkappe 4 weist ferner insbesondere an einem Schnapphaken 4a mindestens einen Schnapper 4b auf, der in eine Lücke zwischen dem Spritzenkörper, insbesondere dessen sich verjüngenden Bereichs, und dem proximalen Ende des rigid needle shield 14 eingreift. Wenn die Abziehkappe 4 von dem Autoinjektor entfernt wird, hakt der Schnapper 4b in das proximale Ende des rigid needle shield 14 ein, wodurch das rigid needle shield 14 von dem Produktbehälter 13 gelöst und zusammen mit der Abdeckkappe 4 von dem Autoinjektor entfernt wird.

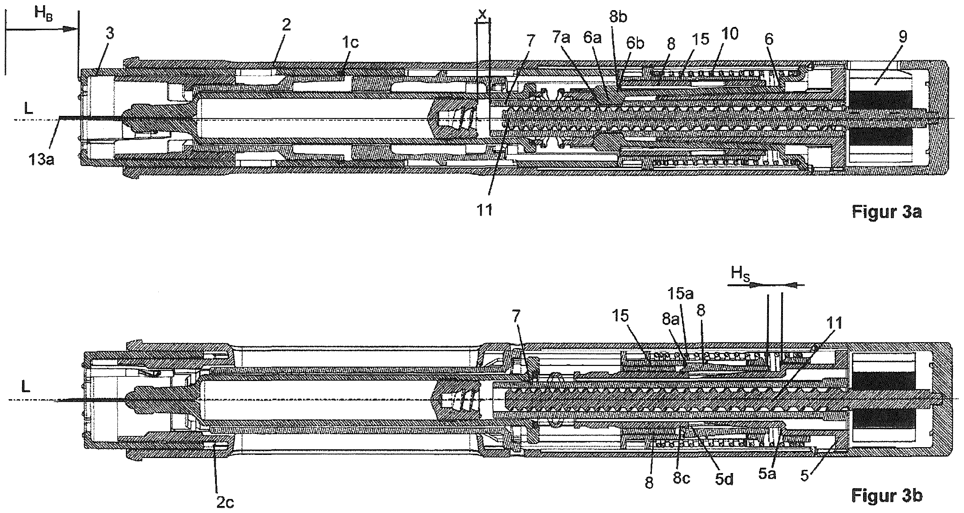

Der Autoinjektor weist eine Nadelschutzhülse 3 auf, die relativ zu dem Gehäuse 2 und entlang der Längsachse L um einen Betätigungshub HB (Fig. 3a, 3b) in die proximale Richtung in eine betätigte Position verschiebbar ist, um eine Produktausschüttung auszulösen. In der Ausgangsposition der Nadelschutzhülse 3, wie sie in den Figuren 2a-2b gezeigt wird, wobei die Abziehkappe 4 abgenommen ist, steht das distale Ende der Nadelschutzhülse 3 distal über die Nadelspitze der Nadel 13a über, so dass ein Zugriff auf die Nadelspitze zunächst verhindert wird. Durch Verschieben der Nadelschutzhülse 3 um den Betätigungshub HB wird die Nadelschutzhülse 3 soweit in die proximale Richtung verschoben, dass die Nadel 13a aus dem distalen Ende der Nadelschutzhülse 3 hervortritt, insbesondere mit einer Länge hervortritt, welche der Injektionstiefe der Nadel in die Einstichstelle entspricht. Bevorzugt soll die Nadel 13a soweit über das distale Ende der Nadelschutzhülse 3 überstehen, dass eine subkutane oder intramuskuläre Injektion erfolgen kann. Insbesondere kann das Gehäuse 2 einen Anschlag 2c (Fig. 3b) bilden, an dem die Nadelschutzhülse 3 in der betätigten Position anliegt.

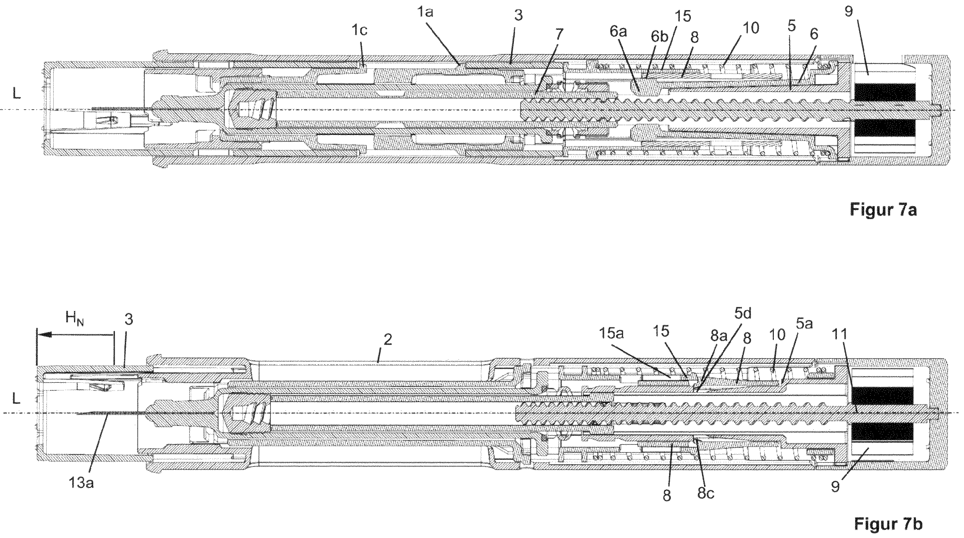

Nach der erfolgten Injektion kann die Nadelschutzhülse 3 relativ zu dem Gehäuse 2 aus der betätigten Position entlang der Längsachse L um einen Nadelschutzhub HN in die distale Richtung in eine Nadelschutzposition (Figuren 7a-7b) verschoben werden. In der Nadelschutzposition steht das distale Ende der Nadelschutzhülse 3 distal über die Nadelspitze über, so dass ein Zugriff auf die Nadelspitze verhindert und eine Verletzungsgefahr verringert wird. Die Nadelschutzhülse 3 kann, wie weiter unten beschrieben wird, gegen erneutes Zurückschieben aus der Nadelschutzposition blockiert werden.

Der Spritzenhalter 1 weist eine Abragung 1a, die radial nach aussen ausgebildet ist, auf, wobei die Abragung 1a in eine schlitzförmige Ausnehmung der Nadelschutzhülse 3, die zwischen dem Gehäuse 2 und dem Spritzenhalter 1 angeordnet ist, eingreift. In der Ausgangsposition der Nadelschutzhülse 3 (Figuren 2a-2b) und/oder in der Nadelschutzposition der Nadelschutzhülse 3 (Figuren 7a-7b) liegt die Nadelschutzhülse 3, insbesondere das proximale Ende der schlitzförmigen Ausnehmung an der Abragung 1a an, wodurch eine Bewegung der Nadelschutzhülse 3 in die distale Richtung verhindert wird. In diese schlitzförmige Ausnehmung, alternativ in eine andere Ausnehmung der Nadelschutzhülse 3, kann ein Nocken 1c, der federnd an dem Spritzenhalter 1 angeordnet und von dem Spritzenhalter 1 gebildet ist, eingreifen. Der Nocken 1c ist so gestaltet, dass bei dem Versuch, die Nadelschutzhülse 3 aus der Ausgangsposition in die betätigte Position zu verschieben, der Nocken 1c die Verschiebung der Nadelschutzhülse 3 zunächst verhindert, wobei der Nocken 1c herausgedrückt wird, wenn die auf die Nadelschutzhülse 3 zum Zurückschieben aufgebrachte Kraft einen gewissen Schwellwert überschreitet, wodurch die Nadelschutzhülse 3 schlagartig in die betätigte Position zurückgeschoben wird. Die Nadel 13a kann hierdurch schlagartig in die Einstichstelle eingestochen werden. Zum Einstechen der Nadel 13a bzw. zum Verschieben der Nadelschutzhülse 3 in die betätigte Position wird das distale Ende der Nadelschutzhülse 3 auf die Einstichstelle angesetzt, wobei das Gehäuse 2 dann in Richtung Einstichstelle gedrückt wird, wobei wenn die Andrückkraft den oben genannten Schwellwert überschreitet, das Gehäuse 2 schlagartig zu der Einstichstelle hin und die Nadelschutzhülse 3 relativ zu dem Gehäuse 2 in die betätigte Position verschoben wird.

Das Gehäuse 2 weist einen ringförmigen Halteabschnitt oder Ringabschnitt 2b auf, der insbesondere das distale Ende des Spritzenhalters 1 insbesondere ringförmig umgibt, und daran anliegt, wodurch die mindestens eine Schulter 1b in dem Eingriff mit dem verjüngenden Abschnitt des Spritzenkörpers gehalten wird. Ferner weist das Gehäuse 2 im Bereich des Halteabschnitts 2b einen Translationsanschlag in der Gestalt einer Halteschulter 2e auf, die verhindert, dass der Spritzenhafter 1 relativ zu dem Gehäuse 2 in die distale Richtung verschiebbar ist, wenn der Spritzenhalter 1 an der Halteschulter 2e anliegt. Dies gilt auch vorteilhaft für die beschriebenen Varianten.

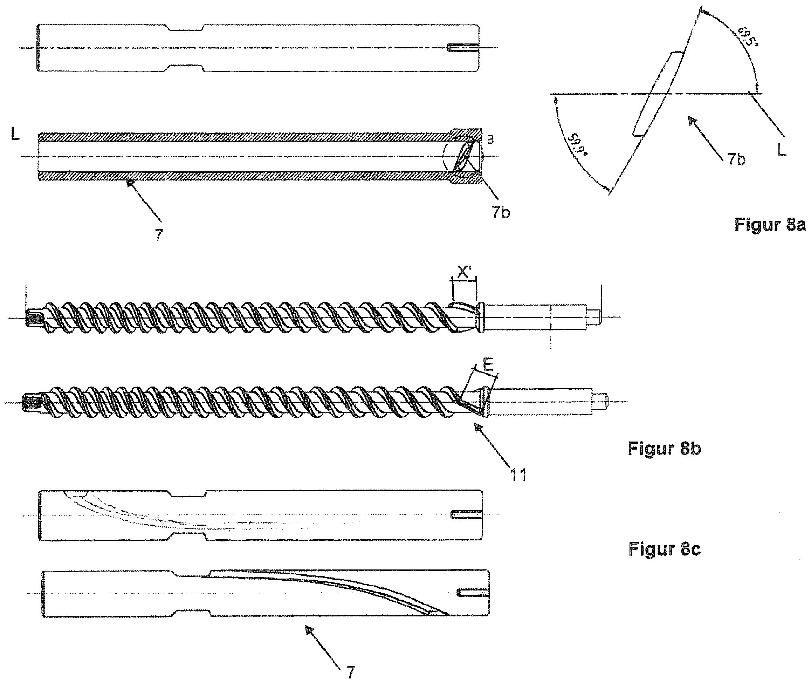

Der Autoinjektor weist ferner ein insbesondere hülsenförmiges Vortriebsglied 7 auf, welches insbesondere an seiner Innenseite einen Gewindeabschnitt insbesondere ein Gewindesegment 7b aufweist (Fig. 8a). das Gewindesegment wird in der Figur 8a in einer abgewickelten Darstellung gezeigt, wobei das Gewindesegment eine insbesondere ovale Form aufweist. Vorzugsweise kann eine Flanke des Gewindesegments 7b des Vortriebsglieds 7 verschiedene Steigungswinkel aufweisen. Wobei bei variabler Gewindesteigung jeweils ein anderer Bereich des Gewindesegments7b vom Gewinde insbesondere einer Gewindestange 11 berührt wird. Insbesondere wir bei einer Schraubbewegung zwischen der Gewindestange 11 und dem mindestens einem Gewindesegment 7b des Vortriebsglieds 7, die Flanke des mindestens einem Gewindesegments 7b an dem Gewinde mit der variablen Steigung der Gewindestange geschraubt, wobei unterschiedliche Bereiche der Flanke das Gewinde mit der variablen Steigung berühren. Das Vortriebsglied ist insbesondere rotativ fest in Bezug auf das Gehäuse.

In der Figur 8c sind weitere bevorzugte Varianten dargestellt. Wie bereits erwähnt kann das Vortriebsglied 7 eine Gewindeverbindung zum Gehäuse oder einem gehäusefesten Element insbesondere dem Mechanikalter aufweisen. Das Gewinde an dem Vortriebsglied 7 kann eine progressive oder degressive Steigung aufweisen. Abhängig davon welches Profil für den Verlauf der Ausschüttkraft erwünscht ist und welche Steigung das Gewinde am Rotationsglied 11 aufweist, wird eine dementsprechende Steigung am Vortriebsglied 7 gewählt.

Bei einem Profil, bei der die Ausschüttkraft konstant sein soll und bei einer kleinen und konstanten Gewindesteigung am Rotationsglied 11, weist das Vortriebsglied eine degressive Steigung auf. D. h. für den Anfangsbereich der Ausschüttung eine kleine Steigung und gegen Ende eine grosse Steigung.

Des Weiteren umfasst der Autoinjektor wie bereits erwähnt ein Rotationsglied (11 insbesondere eine Gewindestange 11 (Fig. 8b), dessen Drehung bewirkt, dass die Federenergie an das Vortriebsglied 7 abgegeben wird, wodurch das Vortriebsglied 7 insbesondere durch einen Gewindetrieb in distale Richtung bewegt wird.

Die Gewindestange 11 ist mit einer ersten Feder 9 verbunden, die die zur Produktausschüttung notwendige Energie speichert und bei Bedarf abgibt. Die Gewindestange 11 ist mit einem Ende der ersten Feder 9 gekoppelt, wobei das andere Ende der ersten Feder 9 mit der Verschlusskappe 12 verbunden ist.

Die Gewindestange 11 weist im gezeigten Beispiel ein Gewinde mit einer variablen Steigung auf, wobei in einem ersten Bereich das Gewinde eine grosse Steigung aufweist. Zwischen der Kolbenstange und dem Kolben ist ein Abstand bzw. Beschleunigungsweg x. Um die Beschleunigung der Kolbenstange auf dem Beschleunigungsweg x zu kontrollieren bzw. abzubremsen, und das Glasbruchrisiko zu minimieren, ist bevorzugt für den Anfang der Kolbenstangenbewegung ein Gewindeeinlaufweg E mit einer grossen Steigung auf der Gewindestange 11 vorgesehen. Bevorzugt ist der Axialabschnitt x' des Gewindeeinlaufwegs E grösser als der Beschleunigungsweg x. Des Weiteren können in einer Lagerposition die Axialkräfte, welche insbesondere durch die Gewindeübersetzung aus dem Drehmoment der Feder entstehen durch eine grosse Steigung an der Gewindestange 11 gering gehalten werden.

Für ein Ausschüttprofil mit einer konstanten Ausschüttkraft variiert das Gewinde bzw. die Gewindesteigung über die Länge der Gewindestange 11. Die Steigung ist degressiv und weist eine immer kleiner werdende Steigung auf, wodurch der Abfall des Federdrehmoments während dem Ausschütten kompensiert werden kann. Wobei die grösste Gewindesteigung im Bereich E nicht selbsthemmend ist.

Die Gewindestange 11 ist axial fest in Bezug auf das Gehäuse 2 und kann sich zumindest in eine, vorzugsweise in distaler Richtung axialfest an dem Mechanikhalter 5 abstützen.

Für die weiter unten beschriebene Erfindung muss die Gewindesteigung auf der Gewindestange nicht zwingend variabel sein. Die hier beschriebene Variante stellt lediglich ein Beispiel dar. Dem Fachmann sind andere Varianten aus dem Stand der Technik bekannt.

Durch die Freigabe des Vortriebsglieds 7 wird der ersten Feder 9 erlaubt, dass sie das Vortriebsglied 7 in distale Richtung bewegt. Die erste Feder 9 ist eine spiralförmige Feder, die im Ausgangs- oder Auslieferungszustand des Autoinjektors mit so viel Energie vorgespannt ist, dass sie das in dem Produktbehälter 13 enthaltene Produkt insbesondere vollständig durch Drehen der Gewindestange 11 und mit Verschieben des Vortriebsglieds 7 um einen Ausschütthub HA aus dem Produktbehälter 13 ausschütten kann. Im Auslieferungszustand der Vorrichtung besteht zwischen dem Kolben 13b und dem distalen Ende des Vortriebsglieds 7 ein Abstand, so dass das Vortriebsglied 7 erst während der Ausführung des Ausschütthubs HA an den Kolben 13b anschlägt und diesen in die Ausschüttrichtung mitnimmt.

Ferner weist der Autoinjektor ein Halteelement 6 auf, welches in diesem Beispiel zwei Arme 6c aufweist, wobei an jedem Arm 6c ein erstes Eingriffselement 6a und ein zweites Eingriffselement 6b angeordnet ist. Das erste Eingriffselement 6a weist radial zu der Längsachse L hin, wobei das zweite Eingriffselement 6b radial von der Längsachse L weg weist. Das erste Eingriffselement 6a greift in eine Ausnehmung 7a, die von dem Vortriebselement 7 gebildet wird, ein, wodurch eine Bewegung des Vortriebsglieds 7 relativ zu dem Halteelement 6 in die distale Richtung oder in die Ausschüttrichtung verhindert wird. Hierdurch wird die erste Feder 9 in ihrem gespannten Zustand gehalten.

Der Autoinjektor weist ein Schaltmodul 8, 15 auf, welches eine Schalthülse 15 und eine von der Schalthülse 15 umgebene Sperrhülse 8 aufweist. Im Auslieferungszustand der Vorrichtung wird das erste Eingriffselement 6a von dem Innenumfang der Sperrhülse 8, der an dem zweiten Eingriffselement 6b anliegt, in dem Eingriff mit der Ausnehmung 7a gehalten.

Die Schalthülse 15 ist mit dem proximalen Ende 3a der Nadelschutzhülse 3 verbunden oder liegt zumindest an dem proximalen Ende 3a der Nadelschutzhülse 3 an. Eine zweite Feder 10, die vorzugsweise die Schalthülse 15 und die Sperrhülse 8 zumindest teilweise umgibt, stützt sich mit ihrem distalen Ende an der Schalthülse 15 ab. Ein Teil der Schalthülse 15 ist somit zwischen der Nadelschutzhülse 3 und dem distalen Ende der zweiten Feder 10 angeordnet. Die zweite Feder 10 ist eine als Druckfeder wirkende und als Wendelfeder ausgestaltete Feder aus Metall. Die zweite Feder 10 stützt sich mit ihrem proximalen Ende an dem Halteelement 6, insbesondere an einer Abragung 6e, die axial verschiebbar und verdrehfest in das Gehäuse 2 eingreift ab. Die zweite Feder 10 umgibt somit auch den Mechanikhalter 4 zumindest teilweise, vorzugsweise vollständig.

Das Schaltglied 15 weist eine Ausnehmung 15a auf, in welche ein Verriegelungsglied 8a der Sperrhülse 8 eingreift. Das Verriegelungsglied 8a ist sägezahnförmig und ragt radial von der Längsachse L weg. Das Verriegelungsglied 8a ist federnd an einem Arm, welcher von der Sperrhülse 8 gebildet wird, angeordnet. Durch Verschieben der Schalthülse 15 in die proximale Richtung wird die Sperrhülse 8 über den Eingriff des Verriegelungsglieds 8a in die proximale Richtung mitgenommen.

Durch Verschieben der Nadelschutzhülse 3 in die betätigte Position wird die Schalthülse 15 ebenfalls um den Betätigungshub HB mitgenommen, wodurch die zweite Feder 10 gespannt wird. Wird die Nadelschutzhülse 3 nicht vollständig in die betätigte Position verschoben, kann die zweite Feder 10 die Schalthülse 15 und die Nadelschutzhülse 3 wieder zurück in die Ausgangsposition verschieben, wobei über den Eingriff des Verriegelungsglieds 8a auch die Sperrhülse 8 von der Schalthülse 15 mitgenommen wird.

Zur Verabreichung des Produkts aus dem Produktbehälter 13 wird die Abziehkappe 4 von dem Autoinjektor zusammen mit dem rigid needle shield 14 entfernt. Das distale Ende der Nadelschutzhülse 3 wird an die Einstichstelle eines Patienten angesetzt, wobei das Gehäuse 2 zu der Einstichstelle hin verschoben wird, wodurch sich die Nadelschutzhülse 3 aus ihrer Ausgangsposition um den Betätigungshub HB in die proximale Richtung relativ zu dem Gehäuse 2 in die betätigte Position bewegt. Hierdurch wird die zweite Feder 10 gespannt, wobei die Schalthülse 15 von der Nadelschutzhülse 3 um den Betätigungshub HB mitgenommen wird. Die Sperrhülse 8 weist eine Ausnehmung bzw. ein distales Ende 8b auf, die durch Verschieben der Sperrhülse 8 um den Betätigungshub HB entlang der Längsachse L auf die Position des zweiten Eingriffselements 6b gebracht wird, wie in den Figuren 3a-3b dargestellt. Hierdurch wird das erste Eingriffselement 6a aus dem Eingriff mit dem Vortriebsglied 7 mit einer Bewegung quer zu und von der Längsachse L weg bewegt, wobei gleichzeitig das zweite Eingriffselement 6b in den Eingriff mit der Sperrhülse 8, insbesondere deren Ausnehmung 8b bewegt wird, Hierdurch wird das Vortriebsglied 7 für die Bewegung um den Ausschütthub HA in die Ausschüttrichtung freigegeben.

Da die axialfeste Kopplung zwischen dem Vortriebsglied 7 und dem Halteelement 6 nun aufgehoben ist, kann das Halteelement 6, welches zumindest ein Stück weit relativ zu dem Gehäuse 2 und entlang der Längsachse L bewegbar ist, von der zweiten Feder 10 in die proximale Richtung bewegt werden, wobei das Halteelement 6 über den Eingriff des zweiten Eingriffselements 6b in die Ausnehmung 8b die Sperrhülse 8 um einen Startsignalhub Hs (Figur 3b) mitnimmt, wodurch die Sperrhülse 8 an einem Startsignalanschlag 5a, der von dem Mechanikhalter 5 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 8 um den Betätigungshub HB wird das Verriegelungsglied 8a und somit auch ein Sperrarm 8c, der an dem federnden Arm der Sperrhülse angebracht ist und sägezahnförmig radial zu der Längsachse L hin ragt, für eine Bewegung quer und zu der Längsachse L hin freigegeben, da der Mechanikhalter 5 eine Vertiefung 5d aufweist, welche eine solche Bewegung des Verriegelungsglieds 8a zulässt, wenn die Sperrhülse 8 um den Betätigungshub HB verschoben wurde oder wenn die Nadelschutzhülse 3 in ihrer betätigten Position ist.

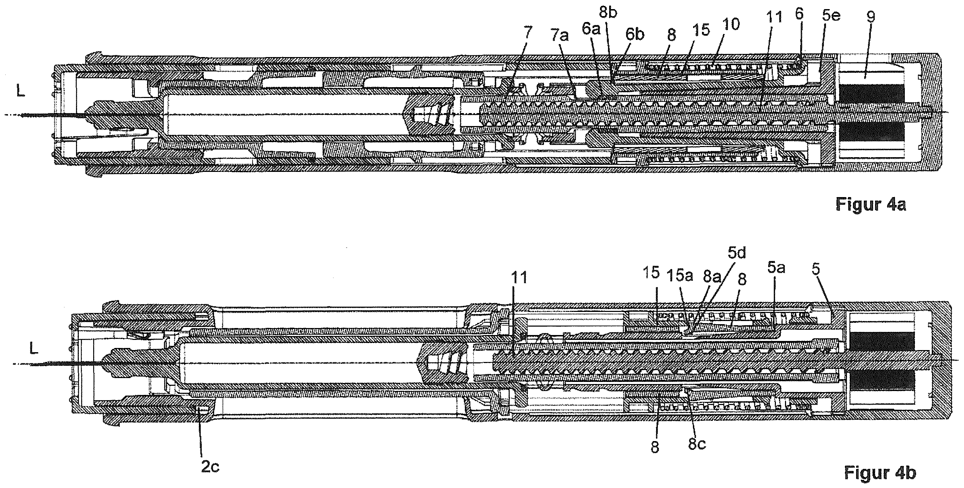

Da das zweite Eingriffsglied 6b immer noch in der Ausnehmung 8b der Sperrhülse 8 ansteht, wird hierdurch das Haltelement 6 daran gehindert, sich weiter in die proximale Richtung relativ zu dem Gehäuse 2 oder der Sperrhülse 8 zu bewegen. Das zweite Eingriffsglied 6b wird von dem Aussenumfang des Vortriebsglieds 7 in den Eingriff mit der Ausnehmung 8b gehalten (Figur 4a), wenn das Vortriebsglied 7 um seinen Ausschütthubs HA bewegt wird.

Am Ende des Ausschütthubs HA gibt das Vortriebsglied 7 das erste Eingriffsglied 6a für eine Bewegung insbesondere zur Längsachse L hin frei, wodurch das zweite Eingriffsglied 6b aus dem Eingriff mit der Ausnehmung 8b der Sperrhülse 8 bewegt wird, so dass die zweite Feder 10 das Halteelement 6 entgegen die Ausschüttrichtung, d. h. in die proximale Richtung beschleunigt, so dass beim Auftreffen des Halteelements 6 auf dem Endsignalanschlag 5e ein akustisches und/oder taktiles Signal erzeugt wird.

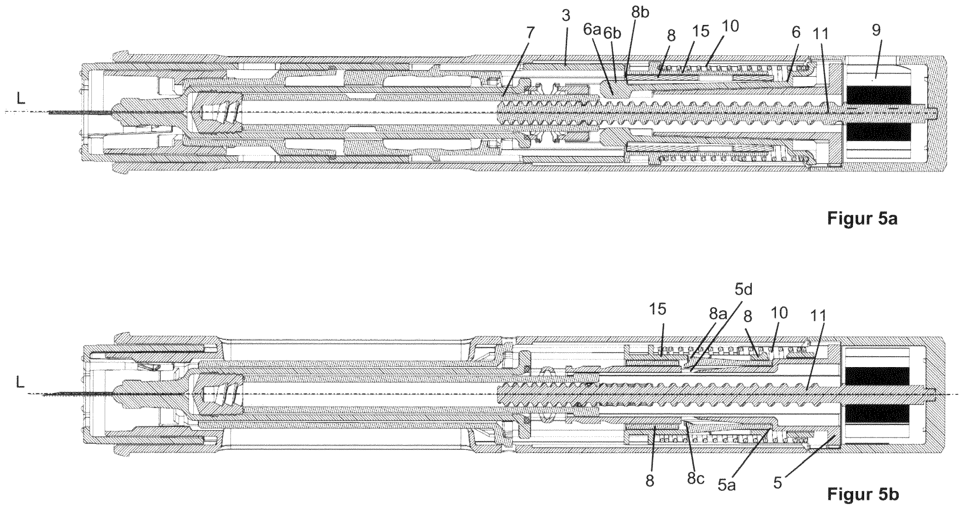

Wie am besten aus Figur 5b erkennbar ist, bleibt der Eingriff des Sperrarms 8c in die erste Ausnehmung 5d vom Mechanikhalter bestehen, wodurch eine Bewegung der Sperrhülse 8 in die distale Richtung relativ zu dem Gehäuse 2 verhindert wird.

Durch Abnehmen des Autoinjektors von der Injektionsstelle kann die zweite Feder 10 die Schalthülse 15 und die Nadelschutzhülse 3 aus der betätigten Position in die Nadelschutzposition um den Nadelschutzhub HN bewegen (Fig. 7a und 7b), wobei das Verriegelungsglied 8a aus dem Eingriff mit der Ausnehmung 15a gedrückt wird, wobei sich die Schalthülse 15 relativ zu der Sperrhülse 8 in die distale Richtung bewegt. Wenn die Nadelschutzhülse 3 in ihrer Nadelschutzposition ist, verschnappt das Verriegelungsglied 8a mit der Schalthülse 15, wobei das Verriegelungsglied 8a ein erneutes Zurückschieben der Nadelschutzhülse 3 in ihre betätigte Position verhindert. Bei dem Versuch, die Nadelschutzhülse 3 aus der Nadelschutzposition in die betätigte Position zurückzuschieben, stösst das Schaltglied 15 an dem Verriegelungsglied 8a an, welches die Bewegung der Nadelschutzhülse 3 in die betätigte Position verhindert. Die Sperrhülse 8 stützt sich hierzu axial an dem Startsignalanschlag 5a des Mechanikhalters 5 ab.

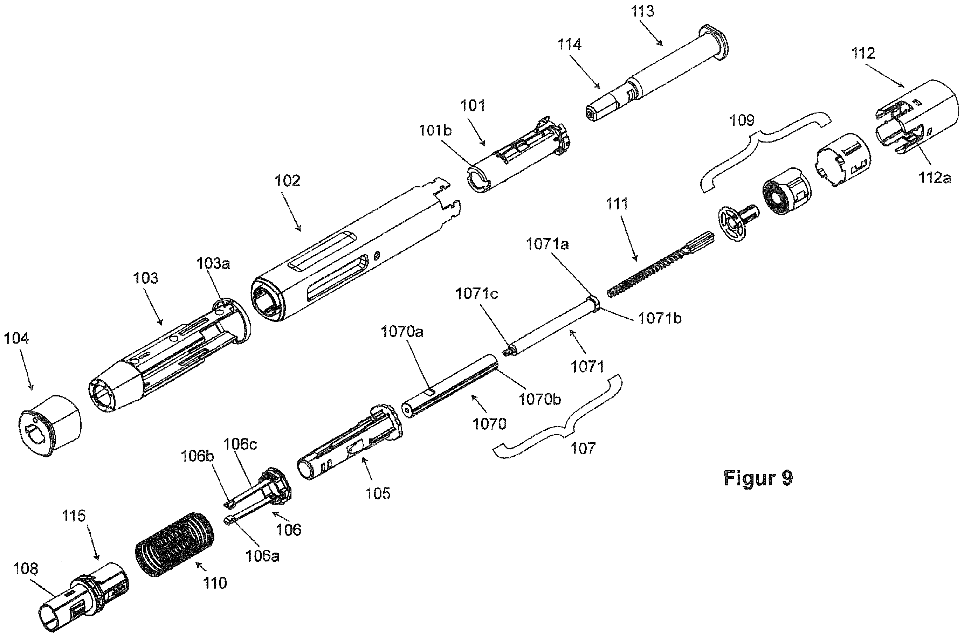

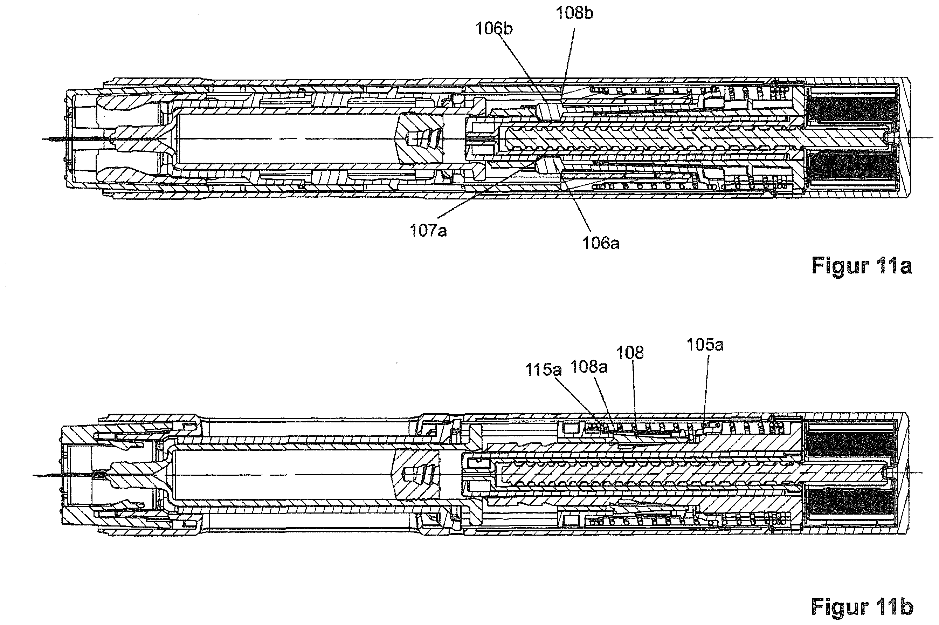

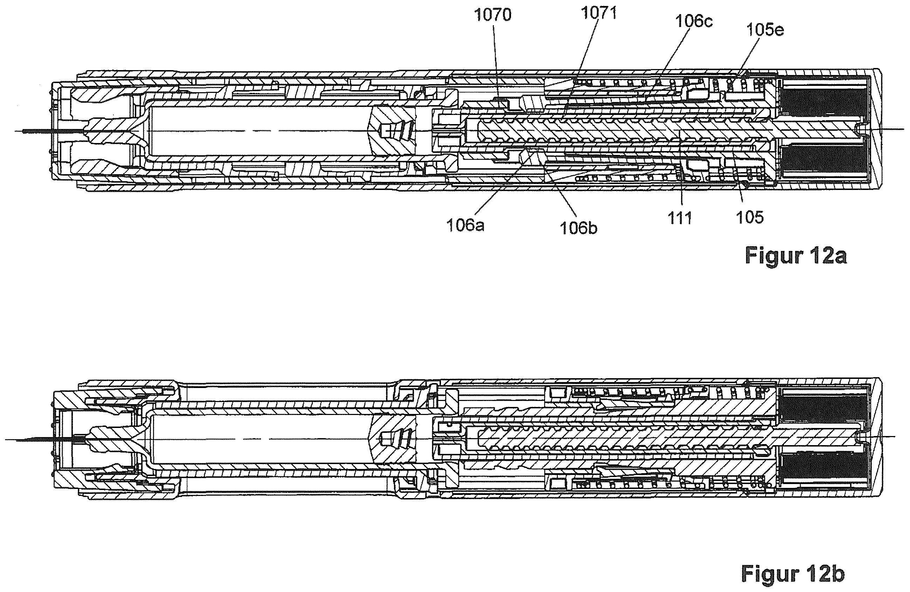

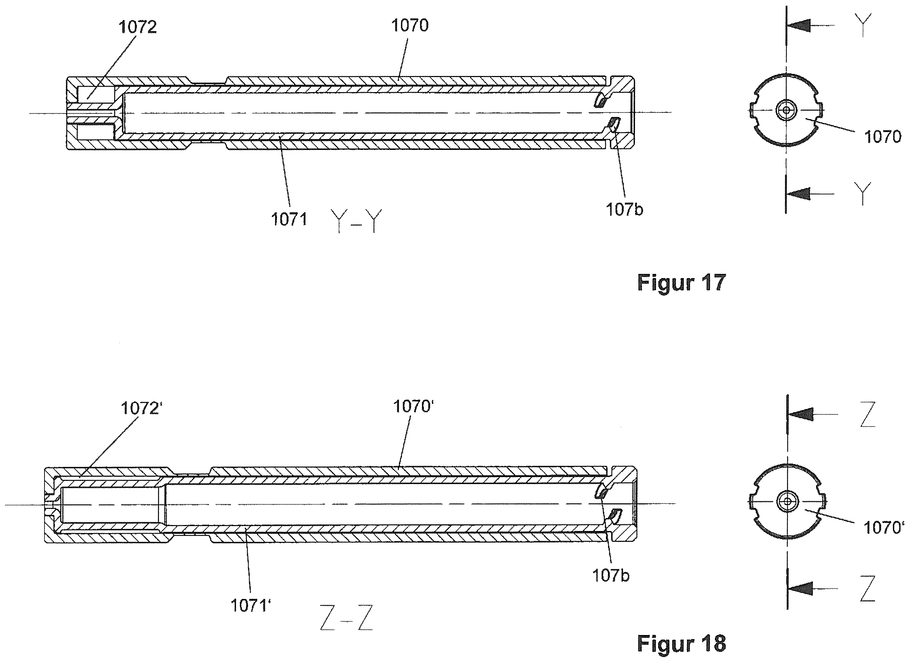

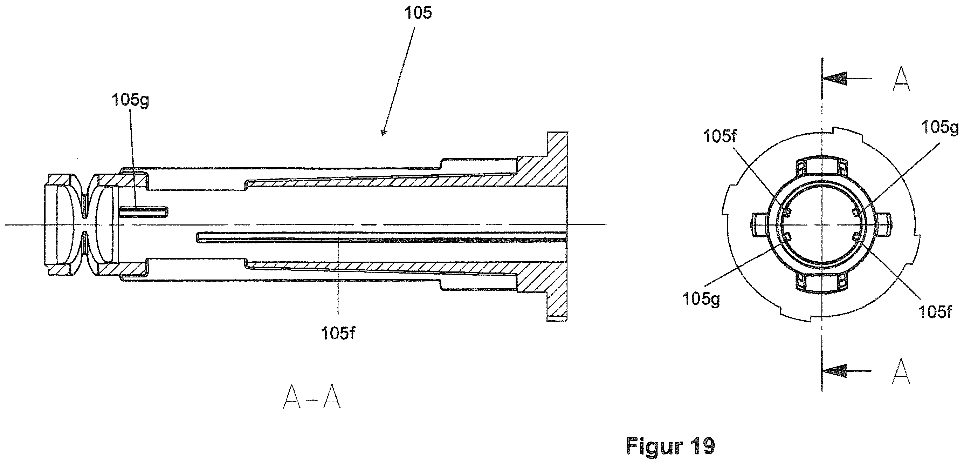

Figuren 9-16 zeigen einen erfindungsgemässen Autoinjektor 100 zuerst in einer Explosionsdarstellung und in verschiedenen Zuständen des funktionellen Ablaufs bei Benutzung. Figuren 17 und 18 zeigen zwei verschiedene erfindungsgemässe Varianten eines mehrteiligen Vortriebsgliedes, welches folgend auch Kolbenstange genannt wird. Figur 19 zeigt einen Schnitt durch eine erfindungsgemässe Ausführung eines Mechanikhalters.

Der erfindungsgemässe Autoinjektor 100 funktioniert grundsätzlich gleich wie der Basis-Autoinjektor 0. Analoge Teile, zum Beispiel der Spritzenhalter, haben Bezeichner, welche sich um die Differenz 100 unterscheiden. Der Spritzenhalter 1 aus dem Basis-Autoinjektor entspricht somit dem Spritzenhalter 101 und so weiter. Da sich auch die grundsätzliche Funktion des Autoinjektors 100 nicht vom Basis-Autoinjektor unterscheidet, wird im Folgenden nur auf die Unterschiede eingegangen.

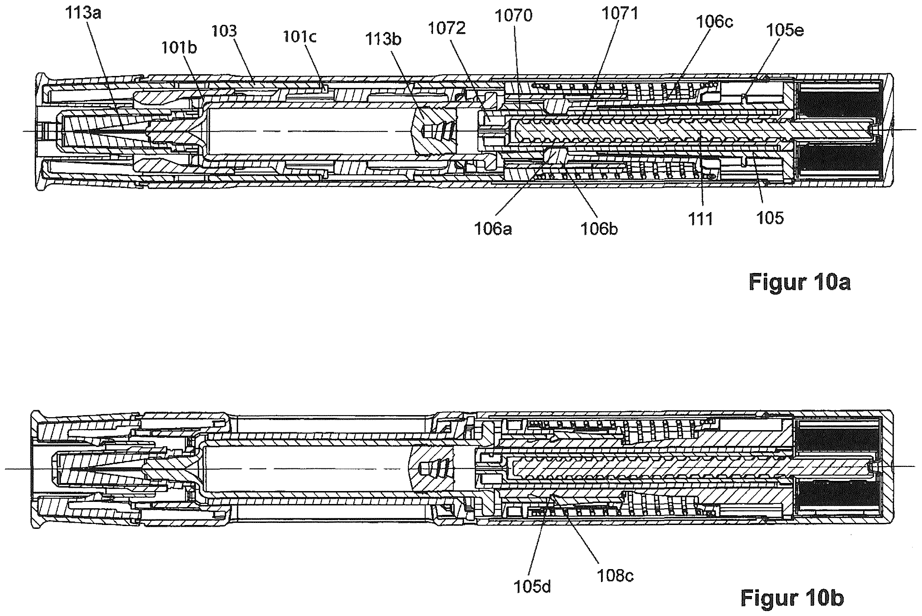

Im Unterschied zum Basis-Autoinjektor 0 ist die Kolbenstange 107 des Autoinjektors 100 zweiteilig ausgestaltet. Die Kolbenstange 107 besteht aus einer äusseren Kolbenstange 1070 und einer inneren Kolbenstange 1071. Beide Teile sind in der Explosionsdarstellung von Figur 9 einzeln dargestellt. Beide Teile sind hülsenförmig ausgestaltet. Wie in Figur 10a zu sehen, ist die innere Kolbenstange 1071 in der äusseren Kolbenstange 1070 gelagert. Das gegenüber dem restlichen Hülsenkörper der inneren Kolbenstange 1071 leicht verdickte proximale Ende 1071a hat in etwa denselben Durchmesser wie die die äussere Kolbenstange 1070 und ist ausserhalb der äusseren Kolbenstange angeordnet. Die innere Kolbenstange 1071 weist eine Gewindeverbindung zur Gewindestange 111 auf.

Die äussere Kolbenstange 1070 ist im Mechanikhalter 105 längsgeführt und verdrehgesichert. Dazu sind auf der äusseren Kolbenstange 1070 ein oder mehrere länglich ausgebildete Führungselemente 1070b angeordnet, die sich in etwa über die ganze Länge der äusseren Kolbenstange 1070 erstrecken. Komplementär dazu sind der Innenfläche des Mechanikhalters 105 Längsführungen 105f und 105g angeordnet, in Figur 19 als Rippen 105f und 105g dargestellt.

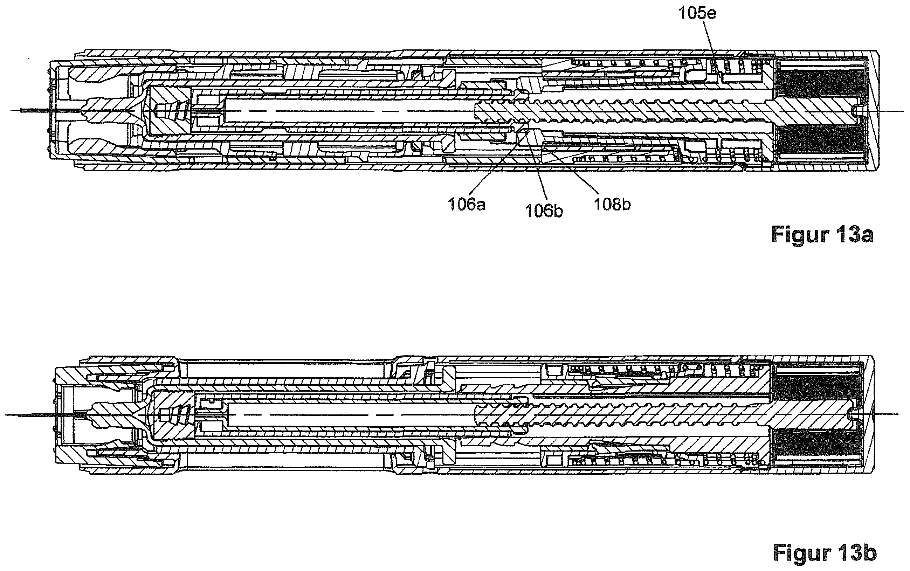

Das proximale Ende 1071 a der inneren Kolbenstange 1071 ist wie beschrieben gegenüber dem restlichen Teil leicht verdickt, so dass eine ringförmige Struktur gebildet wird. Diese ringförmige Struktur wird durch eine oder mehrere Ausnehmungen 1071b unterbrochen - im vorliegenden Beispiel sind es zwei. Diese Ausnehmungen haben zwei Funktionen. Die erste Funktion ist die Verdrehsicherungen zwischen Mechanikhalter 105 (proximale Rippe 105f in Figur 19), die zweite Funktion ist die Freigabe des mindestens einen Halteelements 106 nach Abschluss der Produktausschüttung. Am distalen Ende der inneren Kolbenstange ist eines oder mehrere ruderartige Elemente 1071c angeordnet. Wird die innere Kolbenstange beim Ausschütten von proximal (siehe Figur 10a) um etwa den Ausschütthub nach distal verschoben (Siehe Figur 13a) so ist in der Position der inneren Kolbenstange 1071 in Figur 13a die Verdrehsichung gelöst, weil die Ausnehmungen 1071b in diesem Zustand nicht mehr von den proximalen Rippen 105f geführt werden. Aufgrund der bestehenden Gewindeverbindung der inneren Kolbenstange 1071 mit der Gewindestange 111, auf welche ein Drehmoment aus der Feder 109 wirkt, kann die innere Kolbenstange 1071 sodann in eine Drehung relativ zum Gehäuse 102 und relativ zur äusseren Kolbenstange 1070 versetzt werden.

Betrachtet man den Autoinjektor im Längsschnitt (z. B. in Figur 10a), so sieht man, dass die innere Kolbenstange 1071 und die äussere Kolbenstange 1070 eine Kavität mit einem innerem Volumen 1072 definieren, in welches die ruderartigen Elemente 1071c hineinragen. Dieses Kavität 1072 ist zumindest teilweise mit einem viskosen Fluid, insbesondere einer öligen, zähen, gelartigen oder pastösen Flüssigkeit befüllt (mögliche erfindungsgemässe Varianten des Fluides sind weiter oben beschrieben). Bei einer relativen Rotation zwischen der inneren Kolbenstange 1071 und der äusseren Kolbenstange 1070 das Fluid durch die ruderartigen Elemente 1071c deformiert insbesondere geschert. Das Fluid wirkt als Widerstand gegen die relative Drehung und kann diese Drehung so erfindungsgemäss verzögern. Innere Kolbenstange 1071 und äussere Kolbenstange 1070 bilden zusammen mit dem Fluid eine Verzögerungsvorrichtung

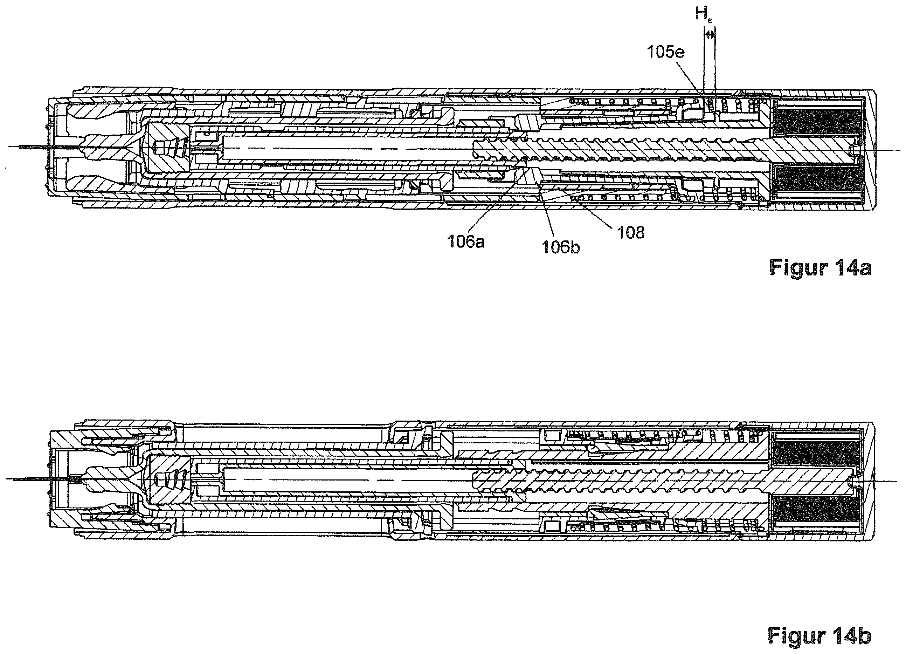

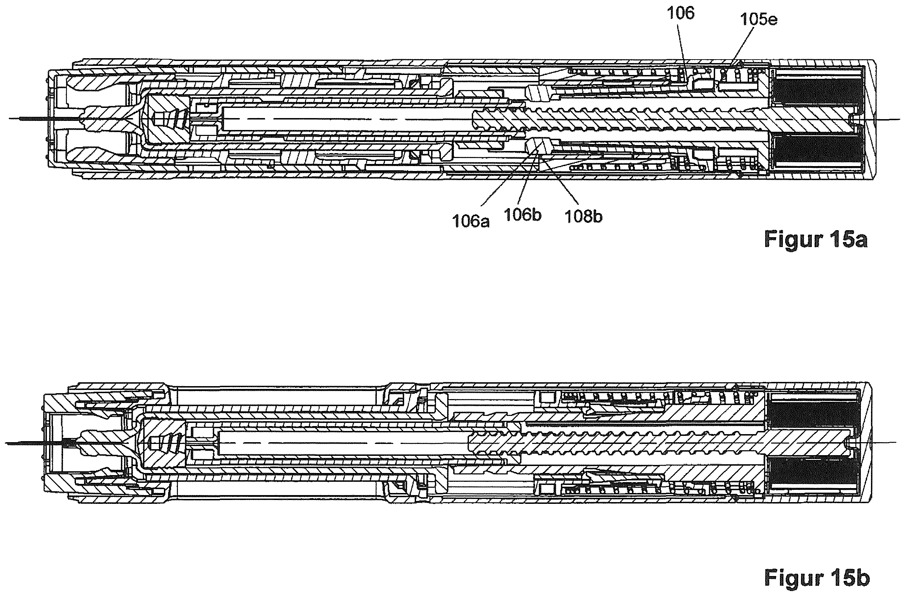

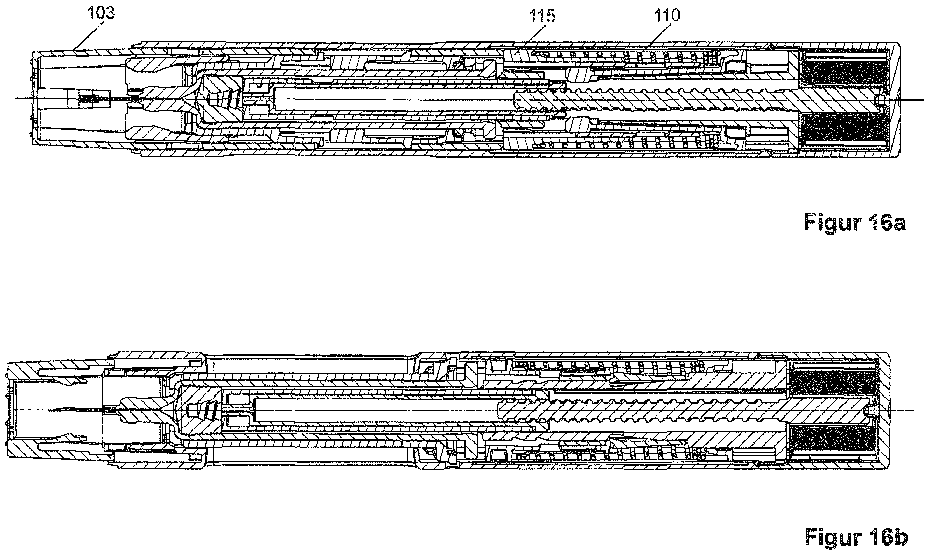

Im Zustand von Figur 13a werden die zweiten Eingriffselement 106b (typischerweise sind es wie in Figur 9 abgebildet zwei solche Elemente) durch das Zusammenwirken von der ringartigen Struktur des proximalen Endes 1071a der inneren Kolbenstange 1071 und den ersten Eingriffselementen 106a in Eingriff mit dem distalen Ende 108b der Sperrhülse 108 gehalten. Da im Zustand von Figur 13a die Verdrehsicherung der inneren Kolbenstange 1071 gelöst ist, beginnt sich diese zu drehen, wodurch auch die Ausnehmungen 1071 b gedreht werden. Erreichen die Ausnehmungen die ersten Eingriffselemente 106a, so kann sich das distale Ende der Arme 106c radial nach innen bewegen, wodurch der Eingriff der zweiten Eingriffselemente 106b und dem distalen Ende 108b der Sperrhülse gelöst wird (siehe Figur 14a/b). Das Halteelement 106 ist somit frei, um sich - angetrieben durch die Feder 110 - in proximale Richtung zu bewegen/beschleunigen, so dass beim Auftreffen des Halteelements 106 auf dem Endsignalanschlag 105e ein akustisches und/oder taktiles Signal erzeugt wird. Figuren 15a/b zeigen den erfindungsgemässen Autoinjektor 100 unmittelbar nach dem Erzeugen des Signals. Das erzeugte Signal signalisiert der benutzenden Person, dass der Autoinjektor 100 von der Injektionsstelle entfernt werden kann. Durch Abnehmen des Autoinjektors 100 von der Injektionsstelle kann die zweite Feder 110 die Schalthülse 115 und die Nadelschutzhülse 1033 aus der betätigten Position in die Nadelschutzposition bewegen wie dies in den Figuren 16a/b gezeigt ist.

Im Folgenden wird die Funktion der Verzögerungsvorrichtung zusammengefasst. Die Ausschüttung von Produkt aus dem Autoinjektor 100 wird gleich wie beim Autoinjektor 0 gestartet und läuft bis vor Ende der Ausschüttung (Fig 13a) auch gleich ab. Im Zustand der Figur 13a besteht eine Verdrehsicherung zwischen äusserer Kolbenstange 1070 und Mechanikhalter 105 während diejenige zwischen innerer Kolbenstange 1071 und Mechanikhalter (wie oben beschrieben) gelöst wird, das über die Gewindestange 111 eingeleitete Drehmoment induziert nun eine Drehung der inneren Kolbenstange 1071 relativ zu Mechanikhalter 105 und äusserer Kolbenstange 1070. Der Drehung entgegen wirkt der durch das viskose Fluid hervorgerufene Widerstand. Dieser Widerstand verzögert die Rotation der inneren Kolbenstange 1071, verhindert sie jedoch nicht. Hat sich die innere Kolbenstange 1071 soweit gedreht, dass die Ausnehmungen 1071b dieselbe Orientierung haben wie die ersten Eingriffselemente 106a, so wird wie beschrieben der Eingriff zwischen den zweiten Eingriffselementen 106b und dem distalen Ende 108b der Sperrhülse 108 gelöst, so dass das gesamte Halteelement für eine axiale Bewegung in proximale Richtung freigegeben ist.

Die Figuren 17 und 18 zeigen zwei verschiedene Ausgestaltungen der Kavitäten 1072 und 1072', welche zwischen innerer Kolbenstange 1071 und äusserer Kolbenstange 1070 (resp. 1070' und 1071') gebildet werden kann. Die Eigenschaften der Verzögerungsvorrichtung können durch die Form der Kavität, die Eigenschaften des Fluids sowie durch zusätzliche Elemente wie ruderartige oder teighakenartige Strukturen auf inneren Oberflächen von innerer oder äusserer Kolbenstange 1070/1071 resp. 107071071' beeinflusst werden. Die Figuren 17 und 18 zeigen lediglich zwei mögliche Ausgestaltungen der Kavität. Während in Figur 17 das Volumen der Kavität am ehesten als zylindrisch beschrieben werden kann, wobei ruderartige Strukturen 1071c in die Kavität eingreifen, um den Bewegungswiderstand zu erhöhen und damit die Verzögerung zu verlängern, so ist die Kavität 1072' in Figur 18 eine Art ringförmiger Spalt. Das Spaltmass kann dabei in einer möglichen Variante soweit verkleinert werden, bis der Fachmann den Spalt als Spiel bezeichnen würde.

### Bezugszeichenliste

- 0: Autoinjektor

- 1: Spritzenhalter
- 1a: Abragung/ Halteglied
- 1b: Schulter/ Eingriffsglied
- 1c: Nocken

- 2: Gehäuse
- 2a: Ausnehmung
- 2b: Ringabschnitt/Halteabschnitt
- 2c: Betätigungsanschlag
- 2e: Halteschulter

- 3: Nadelschutzhülse
- 3a: proximales Ende
- 3b: Schnappgeometrie

- 4: Abziehkappe
- 4a: Schnapphaken
- 4b: Schnapper

- 5: Mechanikhalter
- 5a: Startsignalanschlag
- 5c: Hatlefederabschnitt
- 5d: Vertiefung
- 5e: Endsignalanschlag

- 6: Halteelement
- 6a: erstes Eingriffselement/ glied
- 6b: zweites Eingriffselement/ glied
- 6c: Arm
- 6e: Abragung

- 7: Vortriebsglied
- 7a: Ausnehmung
- 7b: Gewindesegment

- 8: Sperrhülse
- 8a: Verriegelungsglied
- 8b: erste Ausnehmung / distales Ende
- 8c: Sperrarm

- 9: erste Feder/Ausschüttfeder
- 10: zweite Feder/Nadelschutzfeder
- 11: Gewindestange / Rotationsglied

- 12: Verschlusskappe
- 12a: Rastglied
- 13: Produktbehälter/Spritze
- 13a: Nadel
- 13b: Kolben

- 14: rigid needle shield/ Nadelschutzkappe

- 15: Schalthülse
- 15a: Ausnehmung

- 100: Autoinjektor
- 101: Spritzenhalter
- 101a: Abragung/ Halteglied
- 101b: Schulter/ Eingriffsglied
- 101c: Nocken

- 102: Gehäuse
- 102a: Ausnehmung

- 103: Nadelschutzhülse
- 103a: proximales Ende

- 104: Abziehkappe

- 105: Mechanikhalter
- 105a: Startsignalanschlag
- 105d: Vertiefung
- 105e: Endsignalanschlag
- 105f: proximale Längsführung/Rippe
- 105g: distale Längsführung/Rippe

- 106: Halteelement
- 106a: erstes Eingriffselement/ glied
- 106b: zweites Eingriffselement/ glied
- 106c: Arm

- 107: Vortriebsglied/mehrteilige Kolbenstange
- 1070: äussere Kolbenstange
- 1070a: Ausnehmung
- 1070b: Führungselement
- 1071: innere Kolbenstange
- 1071a: proximales Ende

- 1071b: Ausnehmung
- 1071c: ruderartige Elemente
- 107b: Gewindesegment
- 1072: Kavität mit innerem Volumen
- 1072': Kavität mit innerem Volumen

- 108: Sperrhülse
- 108a: Verriegelungsglied
- 108b: erste Ausnehmung / distales Ende
- 108c: Sperrarm

- 109: erste Feder/Ausschüttfeder
- 110: zweite Feder/Nadelschutzfeder

- 111: Gewindestange / Rotationsglied

- 112: Verschlusskappe
- 112a: Rastglied
- 113: Produktbehälter/Spritze
- 113a: Nadel
- 113b: Kolben

- 114: rigid needle shield/ Nadelschutzkappe

- 115: Schalthülse
- 115a: Ausnehmung

- H_{A}: Ausschütthub
- H_{B}: Betätigungshub
- H_{S}: Startsignalhub
- H_{N}: Nadelschutzhub
- Hₑ: Endsignalhub

- L: Längsachse
- E: Gewindeeinlaufweg
- X: Beschleunigungsweg
- X': Axialabschnitt

## Patentansprüche

1. Ausschütteinrichtung (100) zur Verabreichung eines fluiden Produktes, die Ausschütteinrichtung (100) umfassend
- eine Längsachse,
- ein Gehäuse (102) mit einem Mechanikhalter (105), welcher fest mit dem Gehäuse verbunden ist, wobei
- der Mechanikhalter (105) auf einer inneren Oberfläche über mindestens eine Längsführung (105f, 105g) verfügt,
- eine Auslösevorrichtung (106a, 106b, 108),
- einen Produktbehälter (113), insbesondere in der Form einer vorbefüllten Spritze (113) oder Karpule, welcher zumindest axial fest in einem Teil des Gehäuses angeordnet ist, wobei im Produktbehälter (113) ein axial verschiebbarer Stopfen (113b) angeordnet ist, durch dessen Verschiebung in distale Richtung Produkt aus dem Produktbehälter ausgeschüttet werden kann,
eine Triebfeder (109), in welcher Energie für das automatische Ausschütten von Produkt gespeichert ist,
wobei die Triebfeder operativ mit der Auslösevorrichtung (106a, 106b, 108) verbunden ist, und wobei ein erstes Ende der Triebfeder mit dem Gehäuse verbunden ist,
- eine koaxial zur Längsachse angeordnete Gewindestange (111), welche drehfest mit einem zweiten Ende der Triebfeder (109) verbunden ist, wobei die Gewindestange mit einem Drehmoment der Triebfeder beaufschlagt werden kann und in Drehung um die Längsachse versetzt werden kann,
- eine koaxial zur Längsachse angeordnete zwei- oder mehrteilige Kolbenstange (107) mit mindestens einer äusseren Kolbenstange (1070, 1070'), welche die Form einer Hülse hat, welche auf ihrer Aussenfläche mindestens ein erstes Führungselement (1070b) aufweist, das in Eingriff mit der mindestens einen Längsführung (105f, 105g) des Mechanikhalters (105) ist und wobei die äussere Kolbenstange (1070, 1070') mit dem Stopfen (113b) des Produktbehälters so wechselwirken kann, dass bei einer Verschiebung der äusseren Kolbenstange (1070b) in distale Richtung auch der Stopfen (113b) in distale Richtung verschoben werden kann,
die zwei- oder mehrteilige Kolbenstange (107) weiter mit mindestens einer inneren Kolbenstange (1071, 1071'), welche zumindest teilweise im Inneren der äusseren Kolbenstange (1070, 1070') gelagert ist, wobei die innere Kolbenstange (1071, 1071') an ihrem proximalen Ende (1071a), welches über das proximale Ende der äusseren Kolbenstange hinaus ragt, mindestens ein zweites Führungselement aufweist, welches in Eingriff mit der mindestens einen Längsführung (105g, 105f) des Mechanikhalters sein kann, wobei die innere Kolbenstange (1071, 1071') in einer ersten Phase gegenüber dem Mechanikhalter (105) über die Längsführung (105g, 105f) verdrehgesichert und verschiebbar gelagert werden kann und wobei die Verdrehsicherung für eine zweite Phase aufgehoben werden kann und wobei die innere Kolbenstange (1071) über eine Gewindeverbindung (107b, 111) mit der Gewindestange (111) verbunden ist,
- wobei die äussere (1070, 1070') und innere Kolbenstange (1071, 1071') zusammen eine Kavität (1072, 1072') mit einem inneren Volumen definieren, welches zumindest teilweise mit einem viskosen Fluid befüllt ist, so dass insbesondere bei einer relativen Drehung zwischen innerer und äusserer Kolbenstange das viskose Fluid einen Bewegungswiderstand ausbildet, und
- wobei die innere Kolbenstange (1071, 1071') an ihrem proximalen Ende mindestens eine in eine radiale Richtung ragende Freigabestruktur aufweist, insbesondere mindestens eine Vertiefung,
- wobei das Drehmoment in der zweiten Phase eine Drehung der inneren Kolbenstange (1071, 1071') relativ zur äusseren Kolbenstange (1070, 1070') zur Folge hat, welche durch das viskose Fluid gebremst wird und wobei die innere Kolbenstange (1071, 1071') die mindestens eine Freigabestruktur in eine vorbestimmte Orientierung bringen kann, in welcher eine freizugebende Struktur (106a/b) freigegeben werden kann,
- wobei der Bewegungswiderstand dazu verwendet werden kann, eine Rotation der inneren Kolbenstange (1071, 1071') nach dem Lösen der Verdrehsicherung zum Gehäuse (102) zu bremsen, wobei in Folge die Erzeugung eines Endsignals verzögert werden kann.

2. Ausschütteinrichtung nach Anspruch 1, **dadurch gekennzeichnet, dass** das mindestens eine zweite Führungselement und die mindestens eine Freigabestruktur durch eine Ausnehmung (1071b) einer ringförmigen Struktur am proximalen Ende der inneren Kolbenstange (1071) gebildet werden.

3. Ausschütteinrichtung nach Anspruch 1, **dadurch gekennzeichnet, dass** das innere Volumen der Kavität (1072) durch eine lokale und auf dem Umfang verlaufende Verjüngung auf der inneren Kolbenstange (1071') und der der Verjüngung gegenüberliegenden Innenfläche der äusseren Kolbenstange (1070') definiert wird.

4. Ausschütteinrichtung nach Anspruch 1, **dadurch gekennzeichnet, dass** das innere Volumen der Kavität als Spalt zwischen einer äusseren Mantelfläche der inneren Kolbenstange (1071, 1071') und einer inneren Mantelfläche der äusseren Kolbenstange (1070, 1070') definiert wird.

5. Ausschütteinrichtung nach Anspruch 1, **dadurch gekennzeichnet, dass** die Kavität (1072) am distalen Ende der inneren Kolbenstange (1071) lokalisiert ist und in etwa eine zylindrische Form aufweist.

6. Ausschütteinrichtung nach Anspruch 1, **dadurch gekennzeichnet dass** das viskose Fluid eine Newton'sche Flüssigkeit ist.

7. Ausschütteinrichtung nach Anspruch 1, **dadurch gekennzeichnet, dass** das viskose Fluid eine scherverdünnende oder strukturviskose Flüssigkeit ist.

8. Ausschütteinrichtung nach Anspruch 1, **dadurch gekennzeichnet, dass** das viskose Fluid eine thixotrope oder teilthixotrope Flüssigkeit ist.

9. Ausschütteinrichtung nach Anspruch 8, wobei es sich bei der viskosen Flüssigkeit um eine hyaluronsäurehaltige Flüssigkeit handelt.

10. Ausschütteinrichtung nach einem der Ansprüche 1 bis 6, wobei es sich bei der viskosen Flüssigkeit um eine flüssige Silikonformulierung handelt.

11. Ausschütteinrichtung nach Anspruch 1 oder 2, **dadurch gekennzeichnet, dass** das viskose Fluid eine rheopexe oder dilatante Flüssigkeit ist.

12. Ausschütteinrichtung nach Anspruch 11, **dadurch gekennzeichnet, dass** die Flüssigkeit Partikel enthält und/oder eine pastöse Konsistenz aufweist.

13. Ausschüttvorrichtung nach Anspruch 1, die Ausschütteinrichtung (100) weiter umfassend ein Halteelement (106) mit mindestens einem flexiblen Arm (106c) mit einem freien Ende, an welchem die mindestens eine freizugebende Struktur (106a/c) fest angeordnet ist.

14. Ausschüttvorrichtung nach dem vorhergehenden Anspruch, wobei die mindestens eine Freigabestruktur (1071b) eine Vertiefung ist und pro Freigabestruktur mindestens eine freizugebende Struktur vorhanden ist, wobei die mindestens eine freizugebende Struktur als Eingriffselement (106a) ausgebildet ist.

15. Ausschütteinrichtung nach dem vorhergehenden Anspruch, wobei die Ausschüttvorrichtung (100) weiter eine Druckfeder (110) umfasst, welche eine Kraft auf das Haltelement (106) in proximale Richtung ausübt, wobei das Hafteelement (106) ein proximales Ende umfasst und wobei die Freigabe des mindestens einen Eingriffselementes (106a) eine axiale Bewegung des Halteelementes (106) freigibt und durch die wirkende Kraft der Druckfeder (110) eine Bewegung des Halteelementes in axiale Richtung erfolgt.

16. Ausschütteinrichtung nach dem vorhergehenden Anspruch, wobei die Ausschütteinrichtung (100) einen Endanschlag (105e), insbesondere am Mechanikhalter (105), umfasst, welcher die axiale Bewegung des freigegebenen Halteelementes (106) in axiale Richtung begrenzt, so dass im Moment der Begrenzung der Bewegung ein hörbares oder fühlbares Signal erzeugt wird.

## Claims

1. A dispensing device (100) for administering a fluid product, the dispensing device (100) including
- a longitudinal axis,
- a housing (102) having a mechanism holder (105) which is fixedly connected to the housing, wherein
- the mechanism holder (105) has at least one longitudinal guide (105f, 105g) on an inner surface,
- a triggering device (106a, 106b, 108),
- a product container (113), in particular in the form of a pre-filled syringe (113) or carpule which is arranged at least axially fixedly in a part of the housing, wherein arranged in the product container (113) is an axially displaceable plug (113b), by virtue of the displacement of which in the distal direction product can be dispensed from the product container,
- a drive spring (109) in which energy is stored for automatic dispensing of product, wherein the drive spring is operatively connected to the triggering device (106a, 106b, 108) and wherein a first end of the drive spring is connected to the housing,
- a threaded rod (111) which is arranged coaxially with respect to the longitudinal axis and which is non-rotatably connected to a second end of the drive spring (109), wherein the threaded rod can be acted upon with a torque of the drive spring and can be caused to rotate about the longitudinal axis,
- a two-part or multi-part piston rod (107) which is arranged coaxially with respect to the longitudinal axis and has at least one outer piston rod (1070, 1070') which is in the form of a sleeve which on its outer surface has at least one first guide element (1070b) which is in engagement with the at least one longitudinal guide (105f, 105g) of the mechanism holder (105) and wherein the outer piston rod (1070, 1070') can interact with the plug (113b) of the product container in such a way that upon a displacement of the outer piston rod (1070b) in the distal direction the plug (113b) can also be displaced in the distal direction,
the two-part or multi-part piston rod (107) further having at least one inner piston rod (1071, 1071') which is at least partially mounted in the interior of the outer piston rod (1070, 1070'), wherein the inner piston rod (1071, 1071') at its proximal end (1071a) which projects beyond the proximal end of the outer piston rod has at least one second guide element which can be in engagement with the at least one longitudinal guide (105g, 105f) of the mechanism holder, wherein the inner piston rod (1071, 1071') can be mounted displaceably and non-rotatably in a first phase with respect to the mechanism holder (105) by way of the longitudinal guide (105g, 105f) and wherein the rotation-preventing action can be removed for a second phase and wherein the inner piston rod (1071) is connected to the threaded rod (111) by way of a thread connection (107b, 111),
- wherein the outer (1070, 1070') and inner piston rod (1071, 1071') together define a cavity (1072, 1072') with an inner volume which is at least partially filled with a viscous fluid so that in particular upon a relative rotation between the inner and outer piston rod the viscous fluid provides a resistance to movement, and
- wherein the inner piston rod (1071, 1071') at its proximal end has at least one release structure projecting in a radial direction, in particular at least one recess,
- wherein the torque in the second phase results in a rotation of the inner piston rod (1071, 1071') relative to the outer piston rod (1070, 1070') which is braked by the viscous fluid and wherein the inner piston rod (1071, 1071') can bring the at least one release structure into a predetermined orientation in which a structure (106a/b) to be released can be released,
- wherein the resistance to movement can be used to brake a rotation of the inner piston rod (1071, 1071') after release of the rotation-preventing action, wherein as a consequence production of an end signal can be delayed.

2. A dispensing device according to claim 1 **characterised in that** the at least one second guide element and the at least one release structure are formed by an opening (1071b) in an annular structure at the proximal end of the inner piston rod (1071).

3. A dispensing device according to claim 1 **characterised in that** the inner volume of the cavity (1072) is defined by a local tapering extending on the periphery on the inner piston rod (1071') and the inner surface of the outer piston rod (1070') that is opposite the tapering.

4. A dispensing device according to claim 1 **characterised in that** the inner volume of the cavity is defined as a gap between an outer peripheral surface of the inner piston rod (1071, 1071') and an inner peripheral surface of the outer piston rod (1070, 1070').

5. A dispensing device according to claim 1 **characterised in that** the cavity (1072) is located at the distal end of the inner piston rod (1071) and is approximately of a cylindrical shape.

6. A dispensing device according to claim 1 **characterised in that** the viscous fluid is a Newtonian fluid.

7. A dispensing device according to claim 1 **characterised in that** the viscous fluid is a shear-thinning or structural-viscous liquid.

8. A dispensing device according to claim 1 **characterised in that** the viscous fluid is a thixotropic or partially thixotropic liquid.

9. A dispensing device according to claim 8 wherein the viscous liquid is a hyaluronic acid-bearing liquid.

10. A dispensing device according to one of claims 1 to 6 wherein the viscous liquid is a fluid silicone formulation.

11. A dispensing device according to claim 1 or claim 2 **characterised in that** the viscous fluid is a rheopectic or dilatant liquid.

12. A dispensing device according to claim 11 wherein the liquid contains particles and/or is of a pasty consistency.

13. A dispensing device according to claim 1, the dispensing device (100) further including a holding element (106) having at least one flexible arm (106c) having a free end at which the at least one structure (106a/c) to be released is fixedly arranged.

14. A dispensing device according to the preceding claim wherein the at least one release structure (1071b) is a recess and for each release structure there is at least one structure to be released, wherein the at least one structure to be released is in the form of an engagement element (106a).

15. A dispensing device according to the preceding claim wherein the dispensing device (100) further includes a compression spring (110) which exerts a force on the holding element (106) in the proximal direction, wherein the holding element (106) includes a proximal end and wherein the release of the at least one engagement element (106a) enables an axial movement of the holding element (106) and due to the acting force of the compression spring (110) a movement of the holding element in the axial direction takes place.

16. A dispensing device according to the preceding claim wherein the dispensing device (100) includes an end abutment (105e), in particular on the mechanism holder (105), which limits the axial movement of the holding element (106) to be released in the axial direction so that at the moment of limiting the movement an audible or tactile signal is produced.

## Revendications

1. Dispositif de distribution (100) pour l'administration d'un produit fluide, le dispositif de distribution (100) comprenant
- un axe longitudinal,
- un boîtier (102) avec un support mécanique (105), lequel est relié fixement au boîtier, dans lequel
- le support mécanique (105) dispose sur une surface intérieure d'au moins un guidage longitudinal (105f, 105g),
- un dispositif de déclenchement (106a, 106b, 108),
- un contenant de produit (113), en particulier sous la forme d'une seringue (113) préremplie ou carpule, lequel est disposé au moins axialement fixement dans une partie du boîtier, dans lequel un bouchon (113b) mobile axialement, par le déplacement duquel le produit peut être distribué à partir du contenant de produit dans la direction distale, est disposé dans le contenant de produit (113),
un ressort moteur (109), dans lequel est stockée de l'énergie pour la distribution automatique de produit, dans lequel le ressort moteur est relié de manière opérationnelle au dispositif de déclenchement (106a, 106b, 108), et dans lequel une première extrémité du ressort moteur est reliée au boîtier,
- une tige filetée (111) disposée de manière coaxiale par rapport à l'axe longitudinal, laquelle est reliée de manière solidaire en rotation à une deuxième extrémité du ressort moteur (109), dans lequel la tige filetée peut être sollicitée avec un couple du ressort moteur et peut être amenée en rotation autour de l'axe longitudinal,
- une tige de piston en deux parties ou plus (107) disposée de manière coaxiale par rapport à l'axe longitudinal avec au moins une tige de piston extérieure (1070, 1070'), laquelle a la forme d'une douille, laquelle présente sur sa surface extérieure au moins un premier élément de guidage (1070b), qui est en engagement avec l'au moins un guidage longitudinal (105f, 105g) du support mécanique (105) et dans lequel la tige de piston extérieure (1070, 1070') peut interagir avec le bouchon (113b) du contenant de produit, de sorte que lors d'un déplacement de la tige de piston extérieure (1070b) dans la direction distale le bouchon (113b) peut également être déplacé dans la direction distale,
la tige de piston en deux parties ou plus (107) en outre avec au moins une tige de piston intérieure (1071, 1071'), laquelle est montée au moins en partie à l'intérieur de la tige de piston extérieure (1070, 1070'), dans lequel la tige de piston intérieure (1071, 1071') présente à son extrémité proximale (1071a), laquelle fait saillie de l'extrémité proximale de la tige de piston extérieure, au moins un deuxième élément de guidage, lequel peut être en engagement avec l'au moins un guidage longitudinal (105g, 105f) du support mécanique, dans lequel la tige de piston intérieure (1071, 1071') peut être montée de manière bloquée en rotation et de manière mobile dans une première phase par rapport au support mécanique (105) par l'intermédiaire du guidage longitudinal (105g, 105f) et dans lequel le blocage en rotation peut être levé pendant une deuxième phase et dans lequel la tige de piston intérieure (1071) est reliée à la tige filetée (111) par l'intermédiaire d'une liaison filetée (107b, 111),
- dans lequel les tiges de piston extérieure (1070, 1070') et intérieure (1071, 1071') définissent conjointement une cavité (1072, 1072') avec un volume intérieur, lequel est rempli au moins en partie d'un fluide visqueux, de sorte qu'en particulier lors d'une rotation relative entre la tige de piston intérieure et extérieure, le fluide visqueux réalise une résistance au mouvement, et
- dans lequel la tige de piston intérieure (1071, 1071') présente à son extrémité proximale au moins une structure de libération faisant saillie dans la direction radiale, en particulier au moins un évidement,
- dans lequel le couple dans la deuxième phase a pour conséquence une rotation de la tige de piston intérieure (1071, 1071') par rapport à la tige de piston extérieure (1070, 1070'), laquelle est freinée par le fluide visqueux et dans lequel la tige de piston intérieure (1071, 1071') peut amener l'au moins une structure de libération dans une orientation prédéfinie, dans laquelle une structure à libérer (106a/b) peut être libérée,
- dans lequel la résistance au mouvement peut être utilisée pour freiner une rotation de la tige de piston intérieure (1071, 1071') après le déblocage du blocage en rotation par rapport au boîtier (102), dans lequel en conséquence la génération d'un signal de fin peut être retardée.

2. Dispositif de distribution selon la revendication 1, **caractérisé en ce que** l'au moins un deuxième élément de guidage et l'au moins une structure de libération sont formés par un évidement (1071b) d'une structure annulaire à l'extrémité proximale de la tige de piston intérieure (1071).

3. Dispositif de distribution selon la revendication 1, **caractérisé en ce que** le volume intérieur de la cavité (1072) est défini par un amincissement local et s'étendant à la périphérie sur la tige de piston intérieure (1071') et la surface intérieure opposée à l'amincissement de la tige de piston extérieure (1070').

4. Dispositif de distribution selon la revendication 1, **caractérisé en ce que** le volume intérieur de la cavité est défini en tant que fente entre une surface d'enveloppe extérieure de la tige de piston intérieure (1071, 1071') et une surface d'enveloppe intérieure de la tige de piston extérieure (1070, 1070').

5. Dispositif de distribution selon la revendication 1, **caractérisé en ce que** la cavité (1072) est localisée à l'extrémité distale de la tige de piston intérieure (1071) et présente à peu près une forme cylindrique.

6. Dispositif de distribution selon la revendication 1, **caractérisé en ce que** le fluide visqueux est un liquide newtonien.

7. Dispositif de distribution selon la revendication 1, **caractérisé en ce que** le fluide visqueux est un liquide rhéofluidifiant ou pseudoplastique.

8. Dispositif de distribution selon la revendication 1, **caractérisé en ce que** le fluide visqueux est un liquide thixotrope ou en partie thixotrope.

9. Dispositif de distribution selon la revendication 8, dans lequel il s'agit pour le liquide visqueux d'un liquide contenant de l'acide hyaluronique.

10. Dispositif de distribution selon l'une quelconque des revendications 1 à 6, dans lequel il s'agit pour le liquide visqueux d'une formulation de silicone liquide.

11. Dispositif de distribution selon la revendication 1 ou 2, **caractérisé en ce que** le fluide visqueux est un liquide rhéopectique ou rhéoépaississant.

12. Dispositif de distribution selon la revendication 11, **caractérisé en ce que** le liquide contient des particules et/ou une consistance pâteuse.

13. Dispositif de distribution selon la revendication 1, le dispositif de distribution (100) comprenant en outre un élément de retenue (106) avec au moins un bras flexible (106c) avec une extrémité libre, sur laquelle l'au moins une structure à libérer (106a/c) est disposée fixement.

14. Dispositif de distribution selon la revendication précédente, dans lequel l'au moins une structure de libération (1071b) est un évidement et au moins une structure à libérer est présente par structure de libération, dans lequel l'au moins une structure à libérer est réalisée en tant qu'élément d'engagement (106a).

15. Dispositif de distribution selon la revendication précédente, dans lequel le dispositif de distribution (100) comprend en outre un ressort de compression (110), lequel exerce une force sur l'élément de retenue (106) dans la direction proximale, dans lequel l'élément de retenue (106) comprend une extrémité proximale et dans lequel la libération de l'au moins un élément d'engagement (106a) libère un mouvement axial de l'élément de retenue (106) et un mouvement de l'élément de retenue dans la direction axiale s'effectue du fait de la force opérante du ressort de compression (110).

16. Dispositif de distribution selon la revendication précédente, dans lequel le dispositif de distribution (100) comprend une butée de fin de course (105e), en particulier sur le support mécanique (105), laquelle limite le mouvement axial de l'élément de retenue (106) libéré dans la direction axiale, de sorte qu'au moment de la limitation du mouvement, un signal audible ou perceptible est généré.
